# EUROPEAN PATENT APPLICATION

(11) **EP 4 623 805 A1**
(43) Date of publication of application: **01.10.2025**
(21) Application number: 24788863.9
(22) Date of filing: 05.01.2024
(51) Int. Cl.: A61B 5/00, A61B 5/08, A61B 5/05, A61B 5/11, A61B 5/369

(54) **ELECTRONIC APPARATUS AND CONTROL METHOD THEREFOR**

(30) Priority: 10.04.2023 KR 20230046535; 04.09.2023 KR 20230116921
(71) Applicant: Samsung Electronics Co., Ltd., Suwon-si, Gyeonggi-do 16677 (KR)
(72) Inventor: YOON, Daewon, Suwon-si Gyeonggi-do 16677 (KR); PARK, Byeol, Suwon-si Gyeonggi-do 16677 (KR); KIM, Jinhyun, Suwon-si Gyeonggi-do 16677 (KR); BAE, Mugong, Suwon-si Gyeonggi-do 16677 (KR)
(74) Representative: Appleyard Lees IP LLP
(86) International application number: PCT/KR2024/000250
(87) International publication number: WO 2024/214918

(57) **Abstract**

An electronic apparatus includes a communication interface, a microphone, a memory storing at least one instruction, and one or more processors connected to the communication interface, the microphone, and the memory and configured to the electronic apparatus, and the one or more processors are configured to, based on identifying that a user's state corresponds to a sleep state according to a first reflection signal received via the communication interface, not perform a voice recognition function corresponding to a user voice input and perform a sleep recognition function to obtain information corresponding to the sleep state based on the obtained audio, and while the sleep recognition function is being performed, obtain information corresponding to the user's sleep state based on the user state identified by a second reflection signal received via the communication interface and a breathing sound of the user identified by audio obtained via the microphone.

## Description

### [Technical Field]

The disclosure relates to an electronic apparatus and a method for controlling thereof and, more particularly, to an electronic apparatus for obtaining information about a sleep state of a user by sensing a posture of a user and ambient sound and a method for controlling thereof.

### [Background Art]

An electronic apparatus used in everyday life is connected by Internet of Things (IoT) and provides a convenient service customized to a user in real time.

For example, development of an electronic apparatus for performing a customized operation according to a current state of a user by obtaining information about sleep and wake-up states of the user such as a time point at which the user falls into a sleep state, a sleep stage while the user is sleeping, and a time point at which the user wakes up.

A method for sensing the movement and posture of a user via a sensor using transmission/reception of radio waves (or signals) in a space in order to identify the sleep and wake-up state of the user may be used.

In addition thereto, a method of sensing a breathing sound of a sleeping user may be used to identify sleep and wake-up state of a user.

### [Disclosure of Invention]

### [Solution to Problem]

An electronic apparatus according to an embodiment includes a communication interface, a microphone, a memory storing at least one instruction, and one or more processors connected to the communication interface, the microphone, and the memory and configured to the electronic apparatus, and the one or more processors are configured to, based on identifying that a user's state corresponds to a sleep state according to a first reflection signal received via the communication interface, not perform a voice recognition function corresponding to a user voice input and perform a sleep recognition function to obtain information corresponding to the sleep state based on the obtained audio, and while the sleep recognition function is being performed, obtain information corresponding to the user's sleep state based on the user state identified by a second reflection signal received via the communication interface and a breathing sound of the user identified by audio obtained via the microphone.

The one or more processors are configured to obtain first posture information of the user by inputting a first spectrogram obtained from the first reflection signal to a posture state identification model, based on the posture of the user corresponding to the sleep state according to the obtained first posture information of the user, not perform the voice recognition function and perform the sleep recognition function, while performing the sleep recognition function, obtain second posture information of the user by inputting a second spectrogram obtained from the second reflection signal to the posture state identification model, and identify the posture of the user according to the obtained second posture information and identify the user's breathing sound based on audio obtained via the microphone.

The one or more processors are configured to, based on a first movement frequency of the user according to the first reflection signal being less than a first threshold value, not perform the voice recognition function and perform the sleep recognition function, and while performing the sleep recognition function, based on a second movement frequency of the user according to the second reflection signal being less than a first threshold value and the user's breathing sound based on audio obtained via the microphone corresponding the sleep breathing sound, obtain information corresponding to the user's sleep state.

The one or more processors are configured to, based on a first movement radius of the user according to the first reflection signal being less than a second threshold value, not perform the voice recognition function and perform the sleep recognition function, while performing the sleep recognition function, based on the second movement radius of the user according to the second reflection signal being less than a second threshold value and the breathing sound of the user based on the audio obtained via the microphone corresponding to the sleep breathing sound, obtain information corresponding to the user's sleep state.

The one or more processors are configured to, while performing the sleep recognition function, based on the user's posture on the basis of the second reflection signal corresponding to a predetermined posture or receiving a user voice input for a predetermined time or more via the microphone, perform the voice recognition function and not perform the sleep recognition function, and the predetermined posture may include a walking posture, a standing posture, or a sitting posture.

The first reflection signal and the second reflection signal may include information corresponding to the user's posture comprising a walking posture, a standing posture, a sitting posture, a crouching posture, and a lying posture.

Based on the breathing sound corresponding to a sleep breathing sound, the one or more processors may obtain information about the user's sleep stage based on feature information of the sleep breathing sound, and the sleep stage may include at least one of a non-sleep stage, a non-rapid eye movement (REM) sleep stage, and an REM stage.

The one or more processors may identify the breathing sound based on data from which noise is removed, instead of the breathing sound, among audio obtained via the microphone.

The one or more processors may, based on a posture of a user according to the first reflection signal corresponding to a sleep posture, obtain brain wave information of the user, and obtain information corresponding to the sleep state of the user based on the identified posture of the user, the identified breathing sound, and the obtained brain wave information.

The one or more processors may transmit the information corresponding to the obtained sleep state of the user to an external server or an external device connected via the communication interface.

A control method of an electronic apparatus may further include, based on identifying that a user's state corresponds to a sleep state according to a first reflection signal received via the communication interface, not performing a voice recognition function corresponding to a user voice input and performing a sleep recognition function to obtain information corresponding to the sleep state based on the obtained audio; and while the sleep recognition function is being performed, obtaining information corresponding to the user's sleep state based on the user state identified by a second reflection signal and a breathing sound of the user identified by obtained audio.

The performing the sleep recognition function may include obtaining first posture information of the user by inputting a first spectrogram obtained from the first reflection signal to a posture state identification model, based on the posture of the user corresponding to the sleep state according to the obtained first posture information of the user, not performing the voice recognition function and performing the sleep recognition function, and the obtaining the information corresponding to the sleep state of the user may include, while performing the sleep recognition function, obtaining second posture information of the user by inputting a second spectrogram obtained from the second reflection signal to the posture state identification model, and identifying the posture of the user according to the obtained second posture information and identify the user's breathing sound based on obtained audio.

The performing the sleep recognition function may include, based on a first movement frequency of the user according to the first reflection signal being less than a first threshold value, not performing the voice recognition function and performing the sleep recognition function, the obtaining the information corresponding to the sleep state of the user may include, while performing the sleep recognition function, based on a second movement frequency of the user according to the second reflection signal being less than a first threshold value and the user's breathing sound based on audio obtained via the microphone corresponding the sleep breathing sound, obtaining information corresponding to the user's sleep state.

The performing the sleep recognition function may include, based on a first movement radius of the user according to the first reflection signal being less than a second threshold value, not performing the voice recognition function and performing the sleep recognition function, and the obtaining the information corresponding to the sleep state of the user may include, while performing the sleep recognition function, based on the second movement radius of the user based on the second reflection signal being less than a second threshold value and the breathing sound of the user according to the audio obtained via the microphone corresponding to the sleep breathing sound, obtaining information corresponding to the user's sleep state.

The method may further include, while performing the sleep recognition function, based on the user's posture on the basis of the second reflection signal corresponding to a predetermined posture or receiving a user voice input for a predetermined time or more via the microphone, performing the voice recognition function and not performing the sleep recognition function, and the predetermined posture may include a walking posture, a standing posture, or a sitting posture.

The first reflection signal and the second reflection signal may include information corresponding to the user's posture comprising a walking posture, a standing posture, a sitting posture, a crouching posture, and a lying posture.

The obtaining the information corresponding to the user's sleep state may include, based on the breathing sound corresponding to a sleep breathing sound, obtaining information about the user's sleep stage based on feature information of the sleep breathing sound, and The sleep stage may include at least one of a non-sleep stage, a non-rapid eye movement (REM) sleep stage, and an REM stage.

The obtaining the information corresponding to the user's sleep state may include identifying the breathing sound based on data from which noise is removed, instead of the breathing sound, among audio obtained via the microphone.

The obtaining the information corresponding to the user's sleep state may include, based on a posture of a user according to the first reflection signal corresponding to a sleep posture, obtaining brain wave information of the user, and obtaining information corresponding to the sleep state of the user based on the identified posture of the user, the identified breathing sound, and the obtained brain wave information.

The method may include transmitting the information corresponding to the obtained sleep state of the user to an external server or an external device connected via the communication interface.

### [Brief Description of Drawings]

Aspects, features, and advantages of certain embodiments of the disclosure will become more apparent from the description below with reference to the accompanying drawings.
FIG. 1 is a block diagram illustrating a configuration of an electronic apparatus according to an embodiment of the disclosure.
FIG. 2 is a diagram illustrating an operation of each module, which may be included in an electronic apparatus, and interaction between a server and a target application, according to an embodiment of the disclosure.
FIG. 3 is a diagram illustrating an operation in which an electronic apparatus obtains information about a sleep state of a user based on a posture of a user and obtained audio, according to an embodiment of the disclosure.
FIG. 4 is a diagram illustrating a reflected signal and a spectrogram received by an electronic apparatus according to a posture of various users through a communication interface, according to an embodiment of the disclosure.
FIG. 5 is a diagram illustrating a sleep stage of a user, which may be identified by an electronic apparatus, according to an embodiment of the disclosure.
FIG. 6 is a flowchart illustrating an operation in which an electronic apparatus deactivates a voice recognition function and activates a sleep recognition function by identifying a movement frequency of a user, according to an embodiment of the disclosure.
FIG. 7 is a flowchart illustrating an operation in which an electronic apparatus deactivates a voice recognition function and activates a sleep recognition function by identifying a movement radius of a user, according to an embodiment of the disclosure.
FIG. 8 is a block diagram illustrating a configuration of an electronic apparatus according to an embodiment of the disclosure.
FIG. 9 is a flowchart illustrating an operation of an electronic apparatus according to an embodiment of the disclosure.
FIG. 10 is a flowchart illustrating an operation of an electronic apparatus according to an embodiment of the disclosure.
FIG. 11 is a flowchart illustrating an operation of an electronic apparatus according to an embodiment of the disclosure.

### [Mode for Invention]

The disclosure may have various modifications and includes various embodiments, some of which are illustrated in the drawings and described in detail in the detailed description. However, this disclosure is not intended to limit the embodiments described herein but includes various modifications, equivalents, and / or alternatives. In the context of the description of the drawings, like reference numerals may be used for similar components.

In describing the disclosure, well-known functions or constructions are not described in detail since they would obscure the disclosure with unnecessary detail.

In addition, the embodiments described below may be modified in various different forms, and the scope of the technical concept of the disclosure is not limited to the following embodiments. Rather, these embodiments are provided so that this disclosure will be thorough and complete, and will fully convey the scope of the disclosure to those skilled in the art.

The terms used in this disclosure are used merely to describe a particular embodiment, and are not intended to limit the scope of the claims. The expression of a singular includes a plurality of representations, unless the context clearly indicates otherwise.

In this document, the expressions "have," "may have," "including," or "may include" may be used to denote the presence of a feature (e.g., a component, such as a numerical value, a function, an operation, a part, or the like), and does not exclude the presence of additional features.

The expressions "A or B," "at least one of A and / or B," or "one or more of A and / or B," and the like include all possible combinations of the listed items. For example, "A or B," "at least one of A and B," or "at least one of A or B" includes (1) at least one A, (2) at least one B, (3) at least one A and at least one B all together.

In addition, expressions "first", "second", or the like, used in the disclosure may indicate various components regardless of a sequence and/or importance of the components, will be used only in order to distinguish one component from the other components, and do not limit the corresponding components.

It is to be understood that an element (e.g., a first element) is "operatively or communicatively coupled with / to" another element (e.g., a second element) is that any such element may be directly connected to the other element or may be connected via another element (e.g., a third element).

On the other hand, when an element (e.g., a first element) is "directly connected" or "directly accessed" to another element (e.g., a second element), it may be understood that there is no other element (e.g., a third element) between the other elements.

Herein, the expression "configured to" may be used interchangeably with, for example, "suitable for," "having the capacity to," "designed to," "adapted to," "made to," or "capable of." The expression "configured to" does not necessarily mean "specifically designed to" in a hardware sense.

Instead, under some circumstances, "a device configured to" may indicate that such a device can perform an action along with another device or part. For example, the expression "a processor configured to perform A, B, and C" may indicate an exclusive processor (e.g., an embedded processor) to perform the corresponding action, or a generic-purpose processor (e.g., a central processor (CPU) or application processor (AP)) that can perform the corresponding actions by executing one or more software programs stored in the memory device.

The terms such as "module," "unit," "part", and so on are used to refer to an element that performs at least one function or operation, and such element may be implemented as hardware or software, or a combination of hardware and software. Further, except for when each of a plurality of "modules", "units", "parts", and the like needs to be realized in an individual hardware, the components may be integrated in at least one module and be realized in at least one processor.

Various elements and regions in the drawings may be schematically drawn. Accordingly, the technical concept (s) is not limited by a relative size or spacing drawn in the accompanying drawings.

Hereinafter, an embodiment according to the disclosure will be described in detail with reference to the accompanying drawings so as to be easily carried out by a person skilled in the art to which the disclosure belongs.

An electronic apparatus may provide a user with a customized service by obtaining information about a sleep state and sleep stage of a user.

The electronic apparatus may use a method for sensing the movement, posture, or the like of a user via a sensor or a communication interface using transmission/reception of a signal (or radio wave, hereinafter referred to as a signal) in the space in order to identify the sleep and wake-up state of the user.

However, in this case, a sensor or a communication interface should be located at a close distance from a user in order to accurately sense the movement and posture of the user, and thus there is a limitation in sensing the movement and posture of the user due to the restriction of the position of the sensor or communication interface.

Further, an electronic apparatus may use a method of sensing a breathing sound of a sleeping user to identify sleep and wake-up state of a user.

However, in this case, all of audio having a plurality of dimensions, such as amplitude, frequency, and time, obtained via a microphone are analyzed and identified, resulting in a lot of data processing amounts, and thus there is a burden of calculation performance, and uttered voice and noise may be sensed together in addition to the breathing sound of a user, and privacy of a user may be leaked.

Therefore, a method of overcoming a disadvantage of a method of obtaining information about each sleep state and obtaining information about accurate and efficient sleep state is required.

The electronic apparatus according to the disclosure may provide a method of obtaining information about a sleep state of a user by sensing posture of a user and an ambient size.

The electronic apparatus may be, for example, a TV, an air conditioner, a speaker, a computer, a telephone, a lamp, a tablet PC, a smartphone, a display device, a monitor, a projection screen device, a 3D hologram projection device, or the like, but is not limited thereto, and may be various home appliances located around a user outdoors or indoors and used in daily life.

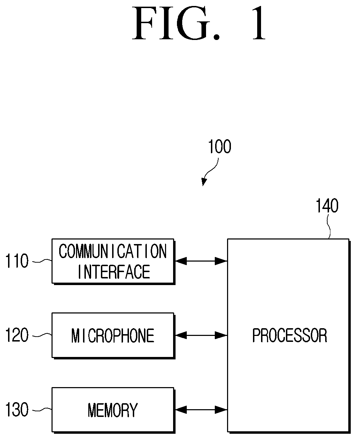

FIG. 1 is a block diagram illustrating a configuration of an electronic apparatus according to an embodiment of the disclosure.

Referring to FIG. 1, an electronic apparatus 100 may include a communication interface 110, a microphone 120, a memory 130, and one or more processors 140.

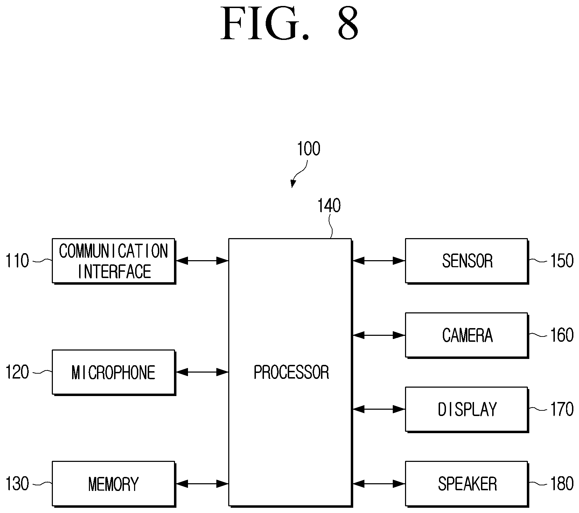

The embodiment is not limited thereto, and a configuration of an apparatus may be further included or a part of a configuration may be omitted as illustrated in FIG. 8 below.

The communication interface 110 may include a wireless communication interface, a wired communication interface, or an input/output interface. The wireless communication method may include, for example, Bluetooth, Bluetooth low energy, CAN communication, Wi-Fi, Wi-Fi Direct, ultrawide band (UWB), ZigBee, Infrared Data Association (IrDA), or Near Field Communication (NFC). The mobile communication technology may include 3GPP, Wi-Max, long term evolution (LTE), fifth generation (5G), but is not limited thereto, and various wireless communication schemes may be utilized.

The wireless communication interface may be implemented by using an antenna that may transmit electromagnetic wave to the outside or receive electromagnetic wave transmitted from the outside, a communication chip, a substrate, or the like.

The electronic apparatus 100 may include only a wireless communication interface or an integrated communication interface that includes or supports both a wireless connection by a wireless communication interface and a wired connection by a wired communication interface.

The electronic apparatus 100 is not limited to a case of including one communication interface 110 for performing one type of communication connection, and may include a plurality of communication interfaces 110 for performing a communication connection in a plurality of ways. Here, the plurality of methods are as described above, but are not limited to the above-described method, and various wireless communication methods may be utilized.

The electronic apparatus 100 performs wireless communication with the external server 200 and a user terminal device via a communication interface to transmit or receive an image, a video, audio, information, or the like, but is not limited thereto. The electronic apparatus 100 includes an input/output interface (not shown) corresponding to a separate HDMI, DP, RGB, DVI, and Thunderbolt method to be connected to an external display device and an external device by wire to transmit or receive an image, a video, audio, information, or the like.

The one or more processors 140 may transmit (or release, hereinafter "transmit") signals to the outside of the electronic apparatus 100 via the communication interface 110. The one or more processors 140 may receive signals reflected or scattered on the surface of an object, such as a human, a wall, a ceiling, furniture, an electronic apparatus, or the like, via the communication interface 110. In addition, the one or more processors 140 may perform communication connection with an external server, an external device, or a user terminal device via the communication interface 110 to transmit/receive various information.

Specifically, the one or more processors 140 may transmit signals such as Wi-Fi, Bluetooth, Radar, IR, microwave, visible rays, or the like, to the outside of the electronic apparatus 100 via the communication interface 110. The one or more processors 140 may simultaneously transmit signals in one or more directions via the communication interface 110.

The one or more processors 140 may receive a reflection signal corresponding to a signal transmitted to the outside of the electronic apparatus via the communication interface 110. At this time, the reflection signal may be a signal reflected or scattered on a surface of an object such as a human, a wall, a ceiling, furniture, an electronic apparatus, or the like.

The one or more processors 140 may identify the difference like amplitude, number of vibrations, wavelength, intensity, or the like, between the signal transmitted to the outside the electronic apparatus 100 and the received signal.

The one or more processors 140 may obtain information about a signal difference, a change according to signal time, or the like based on a signal transmitted to the outside via the communication interface 110 and a signal received via the communication interface 110.

The one or more processors 140 may identify whether the user's posture (e.g., walking posture, standing posture, crouching posture, sitting posture, crouching posture, lying posture, etc.) corresponds to a sleep posture (e.g., lying posture, crouching posture) based on a signal received via the communication interface 110. However, the embodiment is not limited thereto, and the one or more processors 140 may identify the posture of the user based on a sensing result of a separately provided sensor (described below in conjunction with FIG. 8).

The one or more processors 140 perform communication connection with the external server 200, the external device, or the user terminal device via the communication interface 110 to transmit or receive various information, for example, information about the sleep state of the user, posture information of the user, movement information of the user, breathing sound information of the user, brain wave information of the user, an image of the user, or the like.

The one or more processors 140 may perform communication connection with a user terminal device, a remote controller, or the like via the communication interface 110 to receive a signal for controlling the electronic apparatus 100. Here, the user terminal device may be a smartphone, and the one or more processors 140 may receive a signal for a user command input via a control application (e.g., a remote control application) installed in the smartphone via the communication interface 110. Here, the one or more processors 140 may receive a signal for control of the electronic apparatus 100 from the user terminal device via a plurality of communication interfaces 110 (e.g., a Wi-Fi module, a Bluetooth module, etc.) capable of performing different types of communication connections. In this case, the one or more processors 140 may differ in a manner of performing a communication connection with the user terminal device and a method of performing a communication connection with the external server 200, and may perform communication connection with the user terminal device and the external server 200 via different communication interfaces 110.

The communication interface 110 may be included in a presense module. The one or more processors 140 may control the presense module including the communication interface 110 to identify the posture, movement, action, motion, etc. of the user based on the reflection signal received via the communication interface 110.

The embodiment is not limited thereto, and the one or more processors 140 may perform a communication connection with the external server 200, an external device, or a user terminal device via the communication interface 110 to identify a sleep state of the user and transmit or receive various information or control signals necessary for obtaining information about the sleep state of the user.

The microphone 120 may refer to a module for obtaining a sound and converting the sound into an electrical signal, and may be a condenser microphone, a ribbon microphone, a moving coil microphone, a piezoelectric element microphone, a carbon microphone, and a Micro Electro Mechanical System (MEMS) microphone. In addition, the microphone 120 may be implemented in the manner of omnidirectional, bi-directional, unidirectional, sub-cardioid, super cardioid, or hyper cardioid.

The microphone 120 may configure a sound module to identify the breathing sound of a user based on the audio obtained via the microphone 120, but the embodiment is not limited thereto, and the microphone 120 may be implemented with a device configuration separate from the sound module.

While the voice recognition function corresponding to the voice input of the user is being activated, the one or more processors 140 may identify a user voice command based on the user's utterance or voice obtained via the microphone 120. Specifically, the one or more processors 140 may include a speech-to-text (STT) module, and may identify, in a text form, an electrical signal corresponding to the user's utterance or speech via the STT module. The one or more processors 140 may perform an operation corresponding to the identified user voice command. Here, when identifying a user voice command, the one or more processors 140 may identify an electric signal corresponding to a user voice or a user voice command included in a text corresponding to the electrical signal by using a language model (LM) and an automatic sound recognition (ASR) model.

Linguistic understanding is technology of recognizing and applying/processing human language/letter and may include natural language processing, machine translation, dialogue system, question and answer, voice recognition/synthesizing, or the like.

While a sleep recognition function for obtaining information about the sleep state of the user is activated based on the obtained audio, the one or more processors 140 may identify the user's breathing sound based on the audio obtained via the microphone 120. The one or more processors 140 may obtain information about the sleep state of the user based on the identified breathing sound. Specifically, if the identified breathing sound is identified as corresponding to a sleep breathing sound, the one or more processors 140 may identify that the user is in a sleep state.

Further, in a state in which both the voice recognition function and the sleep recognition function are activated, the one or more processors 140 may perform a voice recognition operation for identifying the utterance of the user based on the audio obtained via the microphone 120, or may identify the user's breathing sound to obtain information about the sleep state.

The microphone 120 may be implemented with a plurality of microphones 120 rather than one microphone 120. When the microphone 120 is implemented as a plurality of microphones 120, if the user's posture identified based on the reflection signal received via the communication interface 110 is identified as corresponding to the sleep posture, the one or more processors 140 may stop an audio obtaining operation via some microphones 120. For example, the one or more processors 140 may turn off some microphone 120 and control the power of some microphones 120 to be turned on.

According to various embodiments, when it is identified that the posture of the user corresponds to the sleep posture, the one or more processors 140 may stop the audio processing operation. The one or more processors 140 may obtain audio (or audio information or an audio signal) via the microphone 120. When it is identified that the posture of the user corresponds to the sleep posture, the one or more processors 140 may not process the obtained audio. The one or more processors 140 may not perform a function of processing the obtained audio.

The microphone 120 may be included in a sound module. The one or more processors 140 may control a sound module including the microphone 120 to identify a user's breathing sound based on audio obtained via the microphone 120, or identify whether the identified breathing sound corresponds to a sleep breathing sound.

The memory 130 temporarily or non-temporarily stores various programs or data and transmits the stored information to the one or more processors 140 according to a call of the one or more processors 140. In addition, the memory 130 may store various types of information required for operations, processing, or control operations of the one or more processors 140 in an electronic format.

The memory 130 may include, for example, at least one of a main memory device and an auxiliary memory device. The main memory device may be implemented using a semiconductor storage medium such as a ROM and/or a RAM. The ROM may include, for example, a conventional ROM, an EPROM, an EEPROM, and/or a MASK-ROM. The RAM may include, for example, a DRAM and/or an SRAM. The auxiliary memory device may be implemented using at least one storage medium that may permanently or semi-permanently store data, such as a flash memory device, a secure digital (SD) card, a solid state drive (SSD), a hard disk drive (HDD), a magnetic drum, a compact disc (CD), a digital versatile disc (DVD) or an optical media like a laser disk, a magnetic tape, a magneto-optical disk, and/or a floppy disk.

The memory 130 may store information obtained based on a reflection signal received via the communication interface 110, for example, a motion (e.g., a movement frequency, a movement radius, a movement type, etc.) of a user, a threshold value of a movement radius of the user, a threshold value of a movement frequency of the user, a posture (e.g., a standing posture, a lying posture, a crouching posture, a walking posture, a sitting posture, a sleep posture, etc.), a motion, an operation, or the like. The memory 130 may store a Spectrogram obtained in response to a reflection signal received via the communication interface 110.

The memory 130 may store audio information obtained via the microphone 120. The memory 130 may store audio information obtained by removing noise from audio obtained via the microphone 120. The memory 130 may store information about a user's breathing sound identified based on audio obtained via the microphone 120. The memory 130 may store information about sleep breathing sound corresponding to a sleep stage of a user.

The memory 130 may store information about the identified sleep state of the user based on a reflection signal received via the communication interface 110 or the obtained audio. The memory 130 may store information about a sleep stage of the identified user based on a reflection signal received via the communication interface 110 or the obtained audio.

In addition, the memory 130 may store information required to identify the sleep state of a user, information about the identified sleep state of the user to identify the sleep state of the user based on the reflection signal received via the communication interface 110 or the audio obtained via the microphone 120.

The one or more processors 140 may control the overall operation of the electronic apparatus 100. Specifically, the one or more processors 140 are connected to the configuration of the electronic apparatus 100 including the memory 130 described above, and execute at least one instruction stored in the memory 130 as described above, thereby controlling the operation of the electronic apparatus 100 in an overall manner. In particular, the one or more processors 140 may be implemented as a plurality of one or more processors 140 as well as one or more processors 140.

The one or more processors 140 may be implemented as various methods. For example, the one or more processors 140 may include one or more of a Central Processing Unit (CPU), a Graphics Processing Unit (GPU), an Accelerated Processing Unit (APU), a Many Integrated Core (MIC), a Digital Signal Processor (DSP), a Neural Processing Unit (NPU), a hardware accelerator, or a machine learning accelerator. The one or more processors 140 may control one or any combination of other components of the electronic apparatus 100 and may perform operations or data processing relating to the communication. The one or more processors 140 may execute one or more programs or instructions stored in the memory 130. For example, the one or more processors 140 may perform a method in accordance with one or more embodiments of the disclosure by executing one or more instructions stored in the memory 130.

When a method according to one or more embodiments of the disclosure includes a plurality of operations, a plurality of operations may be performed by one processor 140 or a plurality of processors 140. For example, when a first operation, a second operation, and a third operation are performed by a method according to one or more embodiments, all of the first operation, the second operation, and the third operation may be performed by the first processor, the first operation and the second operation may be performed by a first processor (e.g., a general purpose processor), and the third operation may be performed by a second processor (e.g., an artificial intelligence dedicated processor).

The one or more processors 140 may be implemented as a single core processor including one core, or may be implemented as one or more multicore processors including a plurality of cores (for example, homogeneous multi-cores or heterogeneous multi-cores). When one or more processors 140 is implemented as a multi-core processor, each of the plurality of cores included in the multi-core processor may include a processor internal memory 130 such as an on-chip memory, and a common cache shared by the plurality of cores may be included in the multi-core processor 140. In addition, each of a plurality of cores (or a part of a plurality of cores) included in the multi-core processor may independently read and perform a program command for implementing a method according to one or more embodiments of the disclosure, and may read and perform a program command for implementing a method according to one or more embodiments of the disclosure in connection with all (or a part of) a plurality of cores.

When the method according to one or more embodiments of the disclosure includes a plurality of operations, the plurality of operations may be performed by one core among a plurality of cores included in the multi-core processor or may be performed by the plurality of cores. For example, when a first operation, a second operation, and a third operation are performed by a method according to one or more embodiments, all the first operation, second operation, and third operation may be performed by a first core included in the multi-core processor 140, and the first operation and the second operation may be performed by a first core included in the multi-core processor and the third operation may be performed by a second core included in the multi-core processor.

In the embodiments of the disclosure, the one or more processors 140 may mean a system-on-chip (SoC), a single core processor, a multi-core processor, or a core included in a single core processor or a multi-core processor in which one or more processors and other electronic components are integrated, wherein the core may be implemented as a CPU, a GPU, an APU, a MIC, a DSP, an NPU, a hardware accelerator, or a machine learning accelerator, but embodiments of the disclosure are not limited thereto.

The one or more processors 140 are configured to, based on identifying that a user's state corresponds to a sleep state based on a first reflection signal received via the communication interface 110, not perform a voice recognition function corresponding to a user voice input and perform a sleep recognition function to obtain information corresponding to the sleep state based on the obtained audio, and while the sleep recognition function is being performed, obtain information corresponding to the user's sleep state based on the user state identified by a second reflection signal received via the communication interface 110 and a breathing sound of the user identified by audio obtained via the microphone 120.

The one or more processors 140 may receive a first reflection signal via the communication interface 110. The one or more processors 140 may determine (or obtain) a user state based on the first reflection signal. The one or more processors 140 may identify whether the user state is a sleep state based on the first reflection signal. The first reflection signal may be written as first reflection signal information.

When it is identified that the user state is a sleep state based on the first reflection signal, the one or more processors 140 may not perform a voice recognition function. The one or more processors 140 may deactivate the voice recognition function.

When the user state is identified as a sleep state based on the first reflection signal, the one or more processors 140 may perform a sleep recognition function. The sleep recognition function may include a function of obtaining information corresponding to a sleep state. The one or more processors 140 may obtain audio information via the microphone 120. The one or more processors 140 may obtain information corresponding to the sleep state based on the obtained audio.

While performing the sleep recognition function, the one or more processors 140 may receive a second reflection signal via the communication interface 110. The one or more processors 140 may determine (or obtain) a user state based on the second reflection signal. The one or more processors 140 may identify whether the user state is a sleep state based on a second reflection signal. The second reflection signal may be written as second reflection signal information.

While performing a sleep recognition function, one or more processors 140 may obtain audio information via the microphone 120. The one or more processors 140 may identify a breathing sound of the user based on the obtained audio. The one or more processors 140 may obtain information corresponding to a sleep state of the user based on a user breathing sound.

The information corresponding to the sleep state may include at least one of the information indicating the sleep state or information obtained in the sleep state.

The one or more processors 140 are configured to obtain first posture information of the user by inputting a first spectrogram obtained from the first reflection signal to a posture state identification model, based on the posture of the user corresponding to the sleep state according to the obtained first posture information of the user, not perform the voice recognition function and perform the sleep recognition function, while performing the sleep recognition function, obtain second posture information of the user by inputting a second spectrogram obtained from the second reflection signal to the posture state identification model, and identify the posture of the user according to the obtained second posture information and identify the user's breathing sound based on audio obtained via the microphone 120.

The electronic apparatus 100 may store the state identification model (e.g., neural network model). The electronic apparatus 100 may store the state identification model in the memory 130.

The state identification model may be a model to identify the user's state. The user's state may include at least one of the posture, state of movement, movement frequency, movement radius, or motion type.

The state identification model may include at least one of a posture identification model, a movement identification model, or a motion identification model.

For example, the one or more processors 140 may obtain posture (or posture information) corresponding to the input data as the output data via the posture identification model.

For example, the one or more processors 140 may obtain at least one of the movement state, movement frequency, or movement radius corresponding to the input data as output data via the movement identification model.

For example, the one or more processors 140 may obtain a motion type corresponding to the input data as output data via the motion identification model.

The one or more processors 140 may obtain the first spectrogram from the first reflection signal. The one or more processors 140 may input the first spectrogram as input data to a state identification model. The one or more processors 140 may obtain, as output data, first posture information corresponding to the first spectrogram from the state identification model. Here, the first spectrogram may have different waveforms according to the posture, movement, type of motion, movement frequency, movement radius, or the like of the user. The first posture information may be written as information about the first posture.

The one or more processors 140 may identify the posture of the user based on the first posture information. The one or more processors 140 may determine whether the posture of the user is a sleep posture based on the first posture information. When the posture of the user is determined to be a sleep posture, the one or more processors 140 may not perform a voice recognition function. When the posture of the user is determined to be a sleep posture, the one or more processors 140 may perform a sleep recognition function.

While performing a sleep recognition function, one or more processors 140 may obtain the second reflection signal.

The one or more processors 140 may obtain a second spectrogram from the second reflection signal. The one or more processors 140 may input the second spectrogram as input data to the state identification model. The one or more processors 140 may obtain, as output data, second posture information corresponding to the second spectrogram from the state identification model. Here, the second spectrogram may have different waveforms according to the posture, movement, type of motion, movement frequency, movement radius, or the like of the user. The second posture information may be written as information about the second posture.

The one or more processors 140 may identify the posture of the user based on the second posture information. The one or more processors 140 may obtain audio information via the microphone 120. The one or more processors 140 may identify a breathing sound of the user based on the audio information.

The one or more processors 140 are configured to, based on a first movement frequency of the user based on the first reflection signal being less than a first threshold value, not perform the voice recognition function and perform the sleep recognition function, and while performing the sleep recognition function, based on a second movement frequency of the user based on the second reflection signal being less than a first threshold value and the user's breathing sound based on audio obtained via the microphone 120 corresponding the sleep breathing sound, obtain information corresponding to the user's sleep state.

The one or more processors 140 may obtain (or calculate) a first movement frequency of the user based on the first reflection signal. The one or more processors 140 may compare the first movement frequency and the first threshold value. When the first movement frequency is less than the first threshold value, the one or more processors 140 may not perform a voice recognition function. The one or more processors 140 may deactivate the voice recognition function. When the first movement frequency is less than the first threshold value, the one or more processors 140 may perform a sleep recognition function.

The one or more processors 140 may obtain a second movement frequency of the user based on the second reflection signal. The one or more processors 140 may compare the second movement frequency and the first threshold value. The one or more processors 140 may determine whether the second movement frequency is less than a first threshold value.

The one or more processors 140 may obtain audio information via the microphone 120. The one or more processors 140 may determine whether a breathing sound of a user obtained via audio information corresponds to a sleep breathing sound.

When the second movement frequency is less than a first threshold value and the breathing sound of the user corresponds to the sleep breathing sound, the one or more processors 140 may obtain information corresponding to the sleep state of the user.

The one or more processors 140 are configured to, based on a first movement radius of the user based on the first reflection signal being less than a second threshold value, not perform the voice recognition function and perform the sleep recognition function, while performing the sleep recognition function, based on the second movement radius of the user based on the second reflection signal being less than a second threshold value and the breathing sound of the user based on the audio obtained via the microphone 120 corresponding to the sleep breathing sound, obtain information corresponding to the user's sleep state.

The one or more processors 140 may obtain (or calculate) a first movement radius of the user based on the first reflection signal. The one or more processors 140 may compare the first movement radius and the second threshold value. When the first movement radius is less than the second threshold value, the one or more processors 140 may not perform a voice recognition function. The one or more processors 140 may deactivate the voice recognition function. When the first movement radius is less than the second threshold value, the one or more processors 140 may perform a sleep recognition function.

The one or more processors 140 may obtain a second movement radius of the user based on the second reflection signal. The one or more processors 140 may compare the second movement radius and the second threshold value. The one or more processors 140 may determine whether the second movement radius is less than a second threshold value.

The one or more processors 140 may obtain audio information via the microphone 120. The one or more processors 140 may determine whether a breathing sound of a user obtained through audio information corresponds to a sleep breathing sound.

Based on the second reflection signal being less than a second threshold value and the breathing sound of the user corresponding to the sleep breathing sound, the one or more processors 140 may obtain information corresponding to the user's sleep state.

The one or more processors 140 are configured to, while performing the sleep recognition function, based on the user's posture on the basis of the second reflection signal corresponding to a predetermined posture or receiving a user voice input for a predetermined time or more via the microphone 120, perform the voice recognition function and not perform the sleep recognition function, and the predetermined posture may include a walking posture, a standing posture, or a sitting posture.

The one or more processors 140 may obtain a second reflection signal while performing a sleep recognition function. The one or more processors 140 may determine whether the posture of the user corresponds to a predetermined posture based on the second reflection signal.

If the posture of the user corresponds to a predetermined posture, the one or more processors 140 may perform a voice recognition function. The one or more processors 140 may activate a voice recognition function. When the posture of the user corresponds to a predetermined posture, the one or more processors 140 may not perform a sleep recognition function. The one or more processors 140 may deactivate a sleep recognition function.

The one or more processors 140 may obtain audio information via the microphone 120. The one or more processors 140 may determine whether a user voice input is received for a predetermined time or longer based on the audio information.

When a user voice input is received for more than a predetermined time, one or more processors 140 may perform a voice recognition function. The one or more processors 140 may activate a voice recognition function. When the user voice input is received for more than a predetermined time, the one or more processors 140 may not perform a sleep recognition function. The one or more processors 140 may deactivate a sleep recognition function.

According to various embodiments, when a user's posture corresponds to a predetermined posture and a user voice input is received for more than a predetermined time, one or more processors 140 may activate a voice recognition function and deactivate a sleep recognition function.

According to various embodiments, the predetermined posture may include a walking posture, a standing posture, or a sitting posture.

The first reflection signal and the second reflection signal may include information corresponding to the user's posture comprising a walking posture, a standing posture, a sitting posture, a crouching posture, and a lying posture.

According to various embodiments, the first reflection signal may include information indicating one of a walking posture, a standing posture, a sitting posture, a crouching posture, and a lying posture.

According to various embodiments, the second reflection signal may include information indicating one of a walking posture, a standing posture, a sitting posture, a crouching posture, and a lying posture.

According to various embodiments, the first reflection signal and the second reflection signal may include the same information.

According to various embodiments, the first reflection signal and the second reflection signal may include the different information.

Based on the breathing sound corresponding to a sleep breathing sound, the one or more processors 140 may obtain information about the user's sleep stage based on feature information of the sleep breathing sound, and the sleep stage may include a non-sleep stage, a non-rapid eye movement (REM) sleep stage, and an REM stage.

If the breathing sound corresponds to sleep breathing sound, the one or more processors 140 may obtain feature information of the sleep breathing sound. The one or more processors 140 may obtain information about a sleep stage of the user based on the feature information. The information about the sleep stage may be recited as the sleep stage information. The sleep stage may include at least one of a non-sleep stage, a non-rapid eye movement (REM) sleep stage, and an REM stage.

The information corresponding to the sleep state may include the sleep stage information.

The one or more processors 140 may identify the breathing sound based on data from which noise is removed, instead of the breathing sound, among audio obtained via the microphone 120.

The one or more processors 140 may obtain audio information via the microphone 120. The one or more processors 140 may remove noise included in the audio information. The one or more processors 140 may obtain audio data from which noise has been removed. The one or more processors 140 may identify a breathing sound of the user based on the noise-removed audio data. One or more processors 140 may determine whether a breathing sound of a user corresponds to a sleep breathing sound.

The one or more processors 140 may, based on a posture of a user based on the first reflection signal corresponding to a sleep posture, obtain brain wave information of the user, and obtain information corresponding to the sleep state of the user based on the identified posture of the user, the identified breathing sound, and the obtained brain wave information.

When it is determined that the user's posture is a sleeping posture, the one or more processors 140 may, based on the first reflection signal, obtain brain wave information of the user. The one or more processors 140 may obtain information corresponding to the sleep state of the user based on at least one of the posture of the user, the breathing sound, or the brain wave information.

The one or more processors 140 may transmit the information corresponding to the obtained sleep state of the user to an external server or an external device connected via the communication interface 110.

The one or more processors 140 may transmit the information corresponding to the sleep state to the external server (or external device) via the communication interface 110.

The above-described embodiment describes an operation of deactivating a voice recognition function and performing a sleep recognition function. In the above-described embodiment, an operation of activating a voice recognition function and deactivating a sleep recognition function is described. According to various embodiments, the one or more processors 140 may simultaneously perform a voice recognition function and a sleep recognition function.

The one or more processors 140 perform a voice recognition function and simultaneously perform a sleep recognition function. The one or more processors 140 may perform a voice recognition function and simultaneously perform a sleep recognition function. The one or more processors 140 may activate a sleep recognition function in a state in which a voice recognition function is activated.

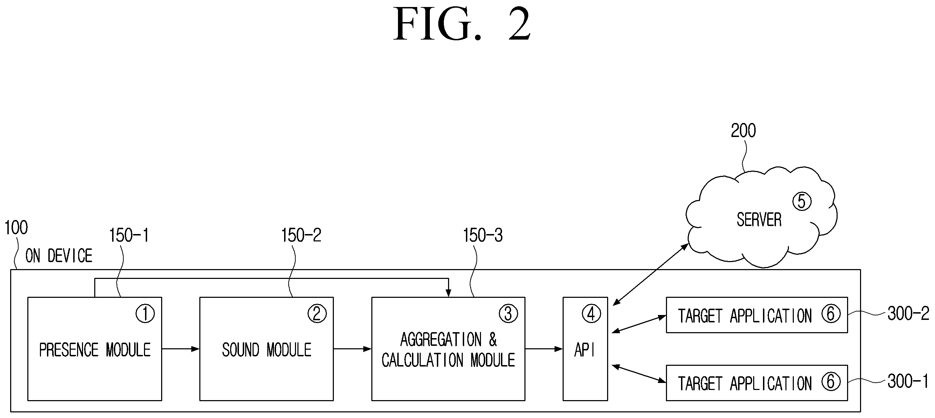

FIG. 2 is a diagram illustrating an operation of each module, which may be included in the electronic apparatus 100, and interaction between a server and a target application, according to an embodiment of the disclosure.

Referring to FIG. 2, the one or more processors 140 may identify whether the user's posture corresponds to the sleep posture based on the first reflection signal received via the communication interface 110.

Here, the one or more processors 140 may control a presense module 150-1 to sense the movement, posture, motion, action, etc. of a user located in a same space with the electronic apparatus 100 or located within a predetermined range from the electronic apparatus 100. In addition, the one or more processors 140 may control the presense module 150-1 to obtain information about a movement (e.g., a movement radius, a movement frequency, a movement type, etc.), a posture (e.g., a standing posture, a lying posture, a sitting posture, a crouching posture, a walking posture, etc.), a motion, and an action of a user based on a reflection signal.

The presense module may include a communication interface 110 (a Wi-Fi module, an IR module, a Bluetooth module, a microwave module, or the like). In addition, the communication interface 110 included in the presense module may use a signal of a frequency band different from a case of performing communication connection with the external server 200 or the external device for a transmitted signal and a received reflection signal when sensing a user's movement, posture or the like.

Without being limited thereto, the one or more processors 140 may receive information obtained by sensing a user's movement, posture, or the like from an external device (e.g., a TV, a display device, a tablet PC, an air conditioner, a lamp, a speaker, a projection screen device, a 3D hologram projection device, a washing machine, a refrigerator, etc.) via the communication interface 110 to identify whether the user's posture is in a sleep state.

When it is identified that the user's posture corresponds to the sleep posture, the one or more processors 140 may deactivate a voice recognition function corresponding to the user voice input and activate a sleep recognition function for obtaining information about the sleep state of the user based on the obtained audio.

The one or more processors 140 may control to stop the operation of at least one module (for example, a pre-processing module, an Automatic Sound Recognition (ASR) module, etc.) among several modules for performing a voice recognition operation in order to deactivate a voice recognition function. At this time, stopping an operation may mean that at least one module (for example, a pre-processing module or an ASR module, etc.) is not loaded into one or more processors 140, but the embodiment is not limited thereto.

In this case, a voice recognition operation with respect to the user's utterance and voice included in the audio obtained via the microphone 120 is not performed and data processing amount may be reduced and power consumption may be reduced.

In addition, the one or more processors 140 perform an operation of identifying a breathing sound of a user included in audio obtained via the microphone 120 to activate a sleep recognition function. Specifically, the one or more processors 140 may increase the sensitivity of the microphone 120 in order to sense a breathing sound of a user. The one or more processors 140 may identify feature information corresponding to a breathing sound of a user included in the obtained audio. The one or more processors 140 may control the sound module to identify feature information corresponding to a breathing sound of a user included in the obtained audio or increase the sensitivity of the microphone 120.

The activation and inactivation of the voice recognition function and the activation and inactivation of the sleep recognition function may be performed based on a control signal received from a wearable device, a smartphone, and a user terminal device.

In addition thereto, the one or more processors 140 may transmit, to the external server 200 or an external device (for example, a TV, an air conditioner, a speaker, a computer, a telephone, a lamp, a tablet personal computer (PC), a smart phone, a display device, a monitor, a projection screen device, a 3D hologram projection device, etc.) information about the first reflection signal about the user's posture, movement, or the like, received via the communication interface 110. The external server 200 or the external device may identify whether the posture of the user corresponds to the sleep posture based on the information about the first reflection signal received from the electronic apparatus 100. When it is identified that the user's posture corresponds to the sleep posture in the external server 200 or the external device, the one or more processors 140 may deactivate the voice recognition function from the external server 200 or the external device via the communication interface 110 and may receive a control signal for activating the sleep recognition function.

Also, the one or more processors 140 may obtain movement, motion, and posture information of a user by capturing the movement of a user via a ToF sensor, an RGB camera, and an IR camera by a projection screen device including the ToF sensor, RGB camera, and IR camera and receiving the sensed result via the communication interface 110.

In addition, one or more processors 140 may receive information about movement, inclination, and relocation of a user terminal device, a remote controller, etc. connected via the communication interface 110, deactivate a voice recognition function and activate a sleep recognition function when it is identified that the user terminal device, the remote controller, or the like does not move, incline, or relocate during a predetermined time.

In addition, the one or more processors 140 may receive, from a wearable device connected via the communication interface 110, for example, lying posture information of the user, heart rate information of the user, brain wave information of the user, or the like. The one or more processors 140 may identify whether the posture of the user corresponds to a sleep posture based on the received lying posture information of the user and the reflection signal received via the communication interface 110. When it is identified that the posture of the user corresponds to the sleep posture, the processor may deactivate the voice recognition function and activate a sleep recognition function.

The one or more processors 140 may identify the user's posture based on the second reflection signal received via the communication interface 110 in a state where the sleep recognition function is activated, and may identify the user's breathing sound based on the audio obtained via the microphone 120.

The one or more processors 140 may, when it is identified that the user is not present in the space based on the second reflection signal received via the communication interface 110, turn off the microphone 120, stop an operation to obtain audio via the microphone 120, or stop the operation to identify the sleep breathing sound.

Here, the one or more processors 140 may control the presense module 150-1 to sense the movement, posture, motion, action, etc. of a user located in a same space with the electronic apparatus 100 or located within a predetermined range from the electronic apparatus 100. In addition, the one or more processors 140 may control the presense module 150-1 to obtain information about the movement, posture, motion, and action of the user of the user based on the reflection signal received via the communication interface 110.

In addition, the one or more processors 140 may control the sound module 150-2 to obtain audio around the electronic apparatus 100 via the microphone 120. The one or more processors 140 may control the sound module 150-2 to identify a breathing sound of the user based on the obtained audio.

Without being limited thereto, the one or more processors 140 may receive information obtained by sensing a user's movement, posture, or the like from an external device (e.g., a TV, a display device, a tablet PC, an air conditioner, a speaker, a light, a projection screen device, a 3D hologram projection device, a washing machine, a refrigerator, etc.), and receive the audio obtained by the external device to identify whether the user's posture is in a sleep state.

The one or more processors 140 may obtain the information about the user's sleep state based on the identified user's posture and the identified breathing sound.

In addition thereto, the one or more processors 140 may identify the breathing sound of the user based on only the audio obtained via the microphone 120 in addition to the reflection signal received via the communication interface 110 in a state in which the sleep recognition function is activated. The one or more processors 140 may identify whether the user is in a sleep state by identifying the posture, movement frequency, movement radius, etc. of the user using only the reflection signal received via the communication interface 110 in addition to the audio obtained via the microphone 120 in a state in which the sleep recognition function is activated. In this case, when it is difficult to identify the sleep state of the user with only the breathing sound of the user, the accuracy of the sleep state determination may be further increased by identifying whether the user is in the sleep state based mainly on the movement and posture of the user.

The one or more processors 140 may assign a weight or a score to each of the reflection signal received via the communication interface 110 and the audio obtained via the microphone 120 to identify the sleep state of the user with a ratio corresponding to the weight or the score to each of the user's posture, the movement reflection signal, and the breathing sound of the user included in the audio.

In addition, when the breathing sound of the user is not identified based on the audio obtained via the microphone 120, the one or more processors 140 may identify the posture and movement of the user based on the reflection signal received via the communication interface 110 to identify whether the posture and movement of the user correspond to the sleep state. The one or more processors 140 may obtain information about the sleep state of the user by identifying whether the identified breathing sound of the user corresponds to the sleep breathing sound based on the audio obtained via the microphone 120 when it is not possible to identify the posture and movement of the user based on the reflection signal received via the communication interface 110.

The one or more processors 140 may identify whether the breathing sound of the user identified based on the personalized breathing sound database corresponds to a sleep breathing sound included in the personalized breathing sound database, to identify a breathing sound of the user. The one or more processors 140 may identify whether a breathing sound of a user of the electronic apparatus 100 corresponds to a personalized breathing sound by inputting the obtained audio to the personalized breathing sound identification model (e.g., neural network model).

The electronic apparatus 100 may include personalized breathing sound database. The personalized breathing sound database may include a breathing sound corresponding to each of a plurality of users. The one or more processors 140 may obtain account information of the electronic apparatus 100.

It is assumed that the user logs into the electronic apparatus 100 by using account information. The one or more processors 140 may obtain account information through a user input.

It is assumed that the user has been automatically logged into the electronic apparatus 100. The one or more processors 140 may obtain the automatically logged-in account information of the electronic apparatus 100.

The account information may include at least one of identification information indicating a specific user and breathing sound information corresponding to a specific user. The one or more processors 140 may obtain specific breathing sound information from the obtained account information. The one or more processors 140 may compare breathing sound information obtained via account information and breathing sound information obtained via audio information. When the breathing sound information obtained via the account information matches the breathing sound information obtained via the audio information, the one or more processors 140 may determine that a breathing sound of the user corresponding to the electronic apparatus 100 account information is identified.

If account information is used, when a plurality of breathing sounds are identified, a breathing sound of a specific user may be easily distinguished. By distinguishing a breathing sound of a person other than the user, an unnecessary control operation may be reduced.

The one or more processors 140 may identify whether all of the plurality of users are in a sleep state by identifying a movement, a posture, or a breathing sound for each of the plurality of users, or identify whether some of the plurality of users are in a sleep state and the remaining part is a non-sleep state.

The one or more processors 140 may input, to a user breathing sound identification model (e.g., a neural network model), information about the waveform of the sleep breathing sound obtained via the microphone 120, and identify whether it is a corresponding breathing sound waveform in the sleep state based on the output vector value.

In addition to the above, the one or more processors 140 may transmit, to the external server 200 or an external device (e.g., a TV, an air conditioner, a speaker, a computer, a telephone, a lamp, a tablet personal computer (PC), a smart phone, a display device, a monitor, a projection screen device, a 3D hologram projection device, etc.) via the communication interface 110, an audio obtained via the microphone 120 and the second reflection signal regarding the user's posture, movement, or the like, received via the communication interface 110 in a state in which the sleep recognition function is activated. Here, a portion corresponding to the user's utterance, user's voice, and a noise (e.g., siren, horn, factory noise, other noise, etc.) among audio obtained via the microphone may be excluded or removed.

To be specific, when at least one of an amplitude, number of vibrations, and a wavelength exceeding a predetermined range in the obtained audio is identified, the one or more processors 140 may identify an audio corresponding to at least one of an amplitude, number of vibrations, and a wavelength exceeding a predetermined range as voice or noise of the user and remove the audio from the obtained audio.

The one or more processors 140 may transmit, to the external server 200 or an external device, the audio from which the user's utterance, voice or noise is removed. The external server 200 or the external device may identify whether the posture of the user corresponds to the sleep posture based on the second reflection signal received from the electronic apparatus 100, and identify the sleep state of the user by identifying a breathing sound of the user based on the audio received from the electronic apparatus 100. In this case, the one or more processors 140 may receive information about the sleep state and the sleep state of the user identified in the external server 200 or the external device via the communication interface 110.

The one or more processors 140 may obtain information about the sleep state by receiving information about a user's sleep state, sleep time of a user, sleep quality of a user, sleep score of a user (proportional to depth of sleep) calculated by the external server 200 or the external device.

In addition, the one or more processors 140 may convert the waveform of audio obtained via the microphone 120 to a spectrogram of an image format and transmit the same to the external server 200. In this case, amount of data for transmission may be reduced and privacy leakage due to transmission of audio as it is may be prevented.

According to various embodiments, one or more processors 140 may obtain audio information (or an audio signal) via the microphone 120. The one or more processors 140 may perform a filtering function with respect to the audio information. The filtering function may include a function of deleting (or removing) data capable of representing personal information. The audio information may be classified into personal information in that the audio information may include a voice print. The one or more processors 140 may obtain filtered audio information by deleting data capable of representing personal information from the audio information. The one or more processors 140 may transmit the filtered audio information to the external server 200.

For example, the filtering function may include a function of removing data corresponding to a predetermined word. The one or more processors 140 may obtain text information based on the audio information. The one or more processors 140 may obtain filtered audio information by removing data (a portion of the audio signal) corresponding to a predetermined word from among the text information.

For example, the filtering function may include a function of removing data corresponding to a predetermined frequency range. The one or more processors 140 may obtain filtered audio information by removing data (a portion of an audio signal) of a predetermined frequency range.

The audio information before filtering may be recited as first audio information and audio information after filtering may be recited as second audio information.

According to various embodiments, one or more processors 140 may use a machine learning model to analyze (or compare) audio information. The machine learning model may include a convolutional neural network (CNN) model. The one or more processors 140 may use a machine learning model including a CNN model in connection with an operation of identifying a breathing sound using audio information.

Here, the one or more processors 140 may control an aggregation & calculation module 150-3 to identify the sleep state of a user based on information about a movement, posture, action, motion, or the like of a user obtained via the presense module 150-1 and a breathing sound of a user identified via the sound module 150-2.

The one or more processors 140 may transmit, to the external server 200, the information about a sleep state of a user by performing communication connection with the external server 200.

The one or more processors 140 may control the execution of the target applications 300-1, 300-2 in the electronic apparatus 100 based on the obtained information about the sleep state. Here, the target application 300-1, 300-2 may be a sleep rhythm measurement application, an alarm application, or the like, but is not limited thereto, and may be an application capable of performing various functions and operations based on the sleep state of a user.

Specifically, the one or more processors 140 may control the speaker 180 to provide an alarm in a predetermined sleep stage or a sleep state so that the user may wake up at a sleep stage or a sleep state where the user feels less fatigue at the time of wake-up based on the sleep stage or the sleep state of the user. In addition, the one or more processors 140 may control the speaker 180 to provide an alarm when it is identified that a user is in a sleep stage where the user may feel less fatigue when waking up, a predetermined sleep stage or sleep stage where the user may wake up in a sleep state, and if it is identified that the time range is within a predetermined time range (for example, five minutes, ten minutes, etc.) from an alarm time set by the user.

In addition, the one or more processors 140 may control the speaker 180 to control provide an alarm in a sleep stage or a sleep state corresponding to a case where sleep satisfaction level is high when the user wakes up, based on the sleep satisfaction information of the user, or variably output an alarm sound.

The one or more processors 140 may control an Internet of Things (IoT) device based on at least one of information about a sleep stage or a sleep state. The IoT device may include a device included in a network to provide various services. The IoT device may include a device for performing a function related to environment control. For example, the IoT device may include at least one of an air conditioner, a lighting, a TV, a refrigerator, a washing machine, a dryer, or a speaker.

The one or more processors 140 may obtain spatial information related to a user. The one or more processors 140 may identify a space in which a user exists among a plurality of spaces based on the location information of the electronic apparatus 100. The one or more processors 140 may identify a target IoT device disposed in a space in which a user is present, from among a plurality of IoT devices. The one or more processors 140 may control the IoT device. For example, if the user identifies that the user is in a sleep state, the one or more processors 140 may control the IoT device to perform a predetermined operation.

For example, the one or more processors 140 may lower the sound (or noise) of the IoT device.

For example, the one or more processors 140 may darken the lighting of the IoT device.

For example, the one or more processors 140 may change a mode of the IoT device to a power-saving mode.

According to various embodiments, the one or more processors 140 may control an IoT device located in a second space, not a first space in which a user is present. When it is identified that the user is in a sleep state, the one or more processors 140 may control the IoT device disposed in the second space. For example, one or more processors 140 may control a central control device or hub device.

According to various embodiments, the one or more processors 140 may transmit a signal for controlling the brightness of the lamp to a lamp via the communication interface 110 based on the obtained information about the sleep stage or the sleep state of the user. Specifically, when it is identified that the user is in a sleep state, a signal for lowering the brightness of the lamp may be transmitted to the lamp via the communication interface 110.

When it is identified that the user is in a sleep state based on the information about the obtained sleep stage or sleep state of the user, the one or more processors 140 may transmit a signal to control power on/off of a TV, a display device, computer or the like to TV, display device, computer via the communication interface 110.

When it is identified that the user is in the sleep state, the one or more processors 140 may raise the set temperature of the air conditioner or a cooling/heating device by a predetermined value or may transmit a control signal to have a predetermined value to the air conditioner or a cooling/heating device via the communication interface 110.

As described above, during ordinary times, the user's posture is sensed only based on a signal having a small data operation capacity without identifying a user's breathing sound, and when it is identified that the posture of the user is a sleep posture according to the reflection signal, the sleep recognition function is activated to sense the posture of the user based on the signal and, at the same time, the breathing sound of the user is identified based on the audio obtained by the microphone 120.

Accordingly, the point in time when the user is changed from non-sleep state to the sleep state may be correctly identified than a case where each of the user's posture sensing or the user's breathing sound sensing is solely used. Also, during the ordinary times, the burden of data throughput may be reduced by performing only the posture monitoring of a user based on a signal without identifying a user's breathing sound. Furthermore, while the sleep recognition function is activated, a voice recognition function for performing a voice recognition operation on the user's utterance or voice is deactivated based on audio obtained via the microphone 120, thereby reducing the burden of performing a large amount of data calculation and preventing privacy leakage.

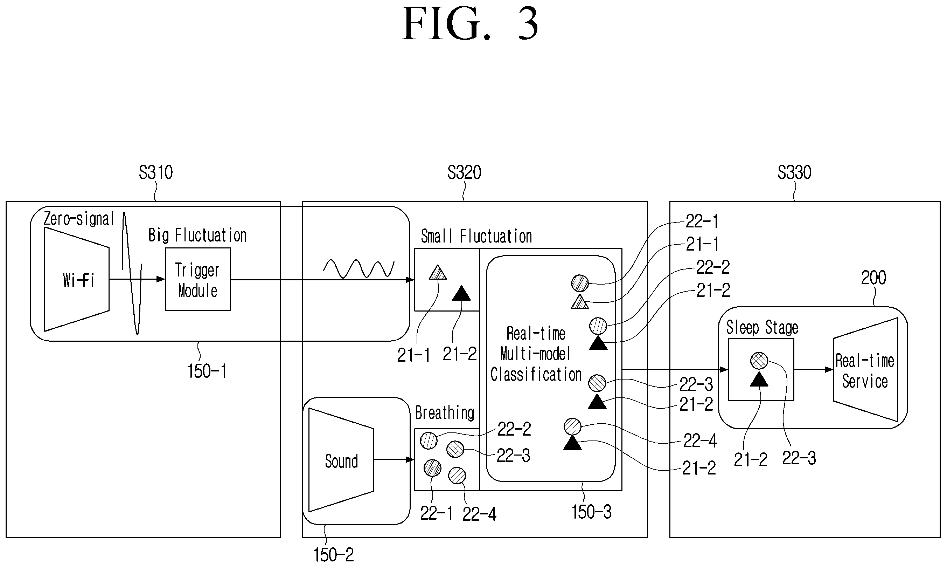

The operation of controlling the configurations of the electronic apparatus 100 by the one or more processors 140 will be further described with reference to FIGS. 3 to 7.

FIG. 3 is a diagram illustrating an operation in which the electronic apparatus 100 obtains information about a sleep state of a user based on a posture of a user and obtained audio, according to an embodiment of the disclosure.

Referring to FIG. 3, the one or more processors 140 may transmit signals such as Wi-Fi, Bluetooth, RADAR, IR, microwave, and visible light to the outside of the electronic apparatus 100, and sense signals such as Wi-Fi, Bluetooth, RADAR, IR, microwave, and visible light reflected and scattered on the inner wall of the space where the electronic apparatus 100 is located, and other furniture, electronic products, and users located around the electronic apparatus 100 via the communication interface 110 in operation S310.

The one or more processors 140 may receive signals outside the electronic apparatus 100 via the communication interface 110. The one or more processors 140 may identify differences such as amplitude, frequency, wavelength, intensity, or the like of a signal transmitted to the outside of the electronic apparatus 100 and a received signal. The one or more processors 140 may obtain information about a signal difference, a change in signal time, or the like based on a signal transmitted to the outside and a signal received via the communication interface 110. The one or more processors 140 may identify the movement of the user based on the Doppler effect of the signal according to the movement of the user, that is, the signal reflected from the distancing object being measured longer than the wavelength, and the signal reflected from the closer object is measured to be shorter in wavelength.

The one or more processors 140 may obtain information about changes in various types of signals according to the posture, action, and movement of a user located in a space. A signal reflection signal having various waveforms may be obtained according to the type, frequency, radius, or the like, of the posture, movement, motion of the user. Here, the user may be located in a same space with the electronic apparatus 100 or may be located at a preset distance from the electronic apparatus 100, but is not limited thereto.

The one or more processors 140 may identify whether the user's posture (e.g., walking posture, standing posture, sitting posture, crouching posture, lying posture) corresponds to the sleep state based on the reflection signal received via the communication interface 110.

The one or more processors 140 may deactivate a voice recognition function corresponding to a user voice input when it is identified that the posture of the user corresponds to a sleep posture based on a first reflection signal received via the communication interface 110, and may activate a sleep recognition function for obtaining information about the sleep state of the user based on the obtained audio.

The one or more processors 140 may deactivate a preprocessing module among several modules performing a voice recognition operation and a part of an encoder and a decoder.

The one or more processors 140 may identify whether the user's posture corresponds to the sleep state based on the vector value output by inputting the second reflection signal received via the communication interface 110 to the state identification model (e.g., neural network model).

As described above, an operation of identifying whether a sensing operation or posture such as a movement, a posture, action, or the like of a user corresponds to a sleep posture based on a reflection signal received via the communication interface 110 may be performed via the presense module 150-1.

In addition, the one or more processors 140 may transmit, to the external server 200 via the communication interface 110, a first reflection signal for the posture, movement, or the like of the user received via the communication interface 110. The external server 200 may identify whether the posture of the user corresponds to the sleep posture based on the first reflection signal received from the electronic apparatus 100. When the external server 200 identifies that the posture of the user corresponds to the sleep posture, the one or more processors 140 may receive a control signal for deactivating a voice recognition function and activating a sleep recognition function from the external server 200 via the communication interface 110.

The one or more processors 140 may obtain a first spectrogram corresponding to the first reflection signal received via the communication interface 110. The one or more processors 140 may obtain first posture information of a user by inputting the obtained first spectrogram into a state identification model (e.g., a neural network model). The first spectrogram may have different waveforms according to a posture, a movement, a type of motion, frequency of movement, radius of movement, or the like.

If it is identified that the user's posture corresponds to the sleep state (e.g., lying posture, crouching posture, etc.), the one or more processors 140 may deactivate the voice recognition function and activate the sleep recognition function.

The one or more processors 140 may deactivate a preprocessing module among several modules performing a voice recognition operation and a part of an encoder and a decoder.

When it is identified that the posture of the user corresponds to a sleep posture (e.g., a lying posture, a crouching posture, etc.), the one or more processors 140 may deactivate a voice recognition function and control a trigger module implemented in the electronic apparatus 100 separately to activate a sleep recognition function. The one or more processors 140 may identify whether the posture of the user corresponds to a sleep posture via the trigger module.

The operation of obtaining the spectrogram corresponding to the reflection signal received via the communication interface 110 of the one or more processors 140 may be performed via the presense module 150-1.

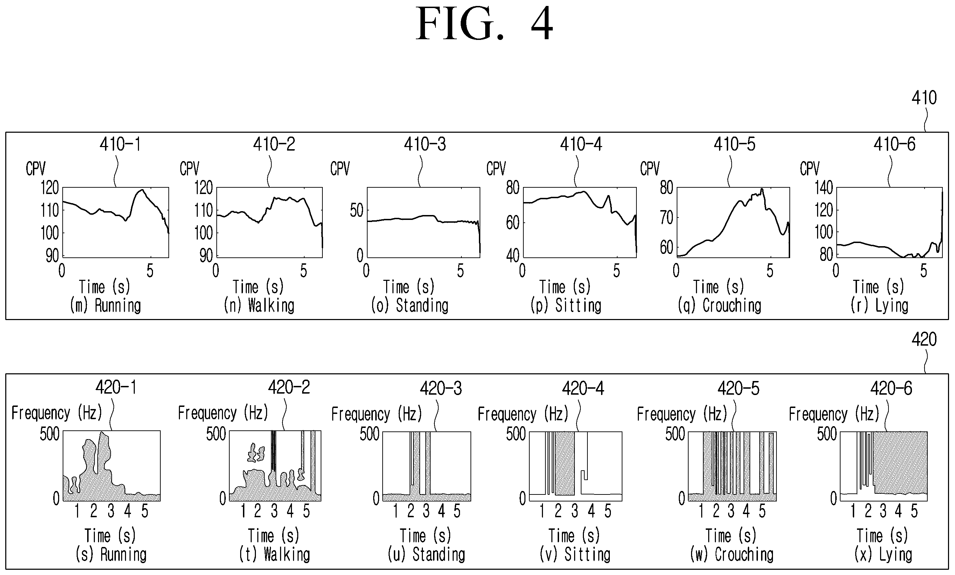

FIG. 4 is a diagram illustrating a reflected signal and a spectrogram received by the electronic apparatus 100 according to a posture of various users through the communication interface 110, according to an embodiment of the disclosure.

Referring to FIG. 4, the one or more processors 140 may obtain the information 410 with respect to the signal change over time based on the reflection signal with respect to the posture, movement, motion, action of the user received via the communication interface 110.

The information 410 with respect to the change in the signal according to time may include signal change information 410-1 for a running posture, signal change information 410-2 with respect to walking posture, signal change information 410-3 for a standing posture, signal change information 410-4 for a sitting posture, signal change information 410-5 for a crouching posture, signal change information 410-6 for a lying posture, or the like, but is not limited thereto.

The one or more processors 140 may obtain the spectrogram 420 corresponding to the user's posture, movement or motion based on the information 410 with respect to the obtained signal change.

The spectrogram 420 may include spectrogram 420-1 corresponding to the running posture, spectrogram 420-2 corresponding to the walking posture, spectrogram 420-3 corresponding to standing posture, spectrogram 420-4 corresponding to sitting posture, spectrogram 420-5 corresponding to crouching posture, and spectrogram 420-6 for lying posture, but is not limited thereto.

The one or more processors 140 may identify the user's posture based on the second reflection signal received via the communication interface 110 in a state where the sleep recognition function is activated, and may identify the breathing sound of the user based on the audio obtained via the microphone 120 in operation S320.

The one or more processors 140 may identify whether the user's posture identified based on the second reflection signal corresponds to a sleep posture (e.g., a lying posture, a crouching posture, etc.). Whether the identified breathing sound corresponds to a sleep breathing sound may be identified based on the audio obtained via the microphone 120.

The one or more processors 140 may identify whether the posture of the user corresponds to a sleep posture based on a vector value output by inputting a second reflection signal received via the communication interface 110 into a state identification model (e.g., a neural network model). The one or more processors 140 may identify whether the breathing sound of the user corresponds to a sleep breathing sound based on a vector value output by inputting, into a breathing sound identification model (e.g., a neural network model), the audio obtained via the microphone 120.

The one or more processors 140 may identify noise, not a breathing sound, from among audio obtained via the microphone 120. The one or more processors 140 may identify noise having characteristics such as amplitude, number of vibrations, wavelength, intensity, and the like of a different type than a breathing sound of a user included in the obtained audio. The one or more processors 140 may identify a breathing sound based on the data where the noise has been removed from the obtained audio.

Here, the one or more processors 140 may control the sound module 150-2 to obtain audio around the electronic apparatus 100 via the microphone 120 or identify a user's breathing sound based on the obtained audio.

In addition, the one or more processors 140 may transmit, to the external server 200 via the communication interface 110, an audio obtained via the microphone 120 and a second reflection signal for the posture, movement, or the like of the user received via the communication interface 110 in a state in which the sleep recognition function is activated. The external server 200 may identify whether the posture of the user corresponds to a sleep posture based on a second reflection signal received from the electronic apparatus 100, and identify a sleep state of the user by identifying a breathing sound of the user based on the audio received from the electronic apparatus 100. In this case, the one or more processors 140 may receive information about the sleep state and the sleep state of the user identified in the external server 200 via the communication interface 110.

In addition, the one or more processors 140 may obtain a second spectrogram corresponding to the second reflection signal in a state in which the sleep recognition function is activated. The one or more processors 140 may obtain second posture information of the user by inputting the obtained second spectrogram into a state identification model (e.g., a neural network model). Here, the second reflection signal and the second spectrogram may have different waveforms according to the posture, movement, type of motion, movement frequency, movement radius, or the like of the user.

The one or more processors 140 may identify the posture of the user based on the obtained second posture information, and identify a breathing sound of the user based on the audio obtained via the microphone 120. Here, the one or more processors 140 may control the sound module 150-2 to obtain audio around the electronic apparatus 100 via the microphone 120 or identify a breathing sound of the user based on the obtained audio.

The second reflection signal has a difference from the first reflection signal in that second reflection signal is a reflection signal in a state where the sleep recognition function is activated and the voice recognition function is deactivated. However, the embodiment is not limited thereto, and the one or more processors 140 may identify the posture of the user based on a first reflection signal received via the communication interface 110 in a state in which the sleep recognition function is activated, and identify a breathing sound of the user based on the audio obtained via the microphone 120.

To be specific, the user's posture identified based on the reflection signal sensed via the communication interface 110 may correspond to a posture 21-1 corresponding to non-sleep state, for example, a standing posture, a walking posture, a running posture, a sitting posture, or a posture 21-2 corresponding to a sleep state, for example, a lying posture or a crouching posture.

The user's breathing sound identified based on the audio obtained via the microphone 120 may correspond to a breathing sound 22-1 in a non-sleep state, a breathing sound 22-2 of a first non-REM state, a breathing sound 22-3 of a second non-REM state, and a breathing sound 22-4 of a REM state.

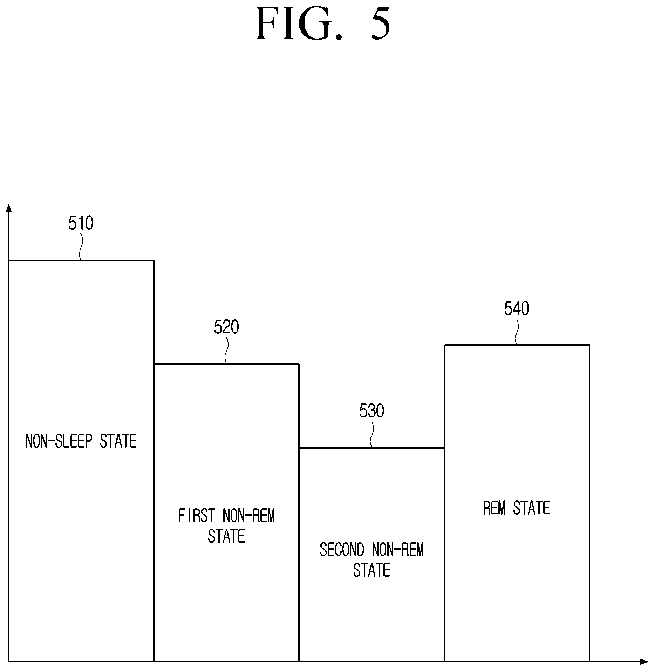

FIG. 5 is a diagram illustrating a sleep stage of a user, which may be identified by the electronic apparatus 100, according to an embodiment of the disclosure.

Referring to FIG. 5, the sleep state or sleep stage of the user may be divided into several steps. Specifically, the sleep state or sleep stage of the user may include a non-sleep state 510, a first non-REM state 520, a second non-REM state 530, and a REM state 540. In the coordinate system of FIG. 5, a vertical axis may correspond to a level of awakeness, and a horizontal axis may correspond to a time axis, but is not limited thereto.

The non-sleep state 510 may mean a complete awake state where a user does not fall asleep or half-awake state.

The first non-REM state 520 and the second non-REM state 530 may be a sleep state, not the REM state. Each sleep state that is non-REM may have several stages or several types, different types of brainwaves may be observed for each non-REM state, and quality and depth of sleep may be different for each sleep state or sleep stage.

The REM stage 540 refers to sleep of a stage where rapid moving of eyeball for several times is observed among stages of sleep. The brainwave of a person in the REM state 540 may have a similarity with non-sleep state, and may have a somewhat higher level of awakeness than the non-REM 520, 530.

The one or more processors 140 may obtain information about the sleep state of the user based on the identified user's posture and the identified breathing sound.

To be specific, the one or more processors 140 may, when the identified user's posture is a posture 21-1 corresponding to the non-sleep state, and the identified breathing sound of the user is a breathing sound 22-1 of the non-sleep state, identify that the user is in the non-sleep state.

The one or more processors 140 may, if the identified user's posture is a posture 21-2 corresponding to the sleep state, and the identified user's breathing sound is a breathing sound 22-2, 22-3, 22-4 in the sleep state, identify that the user is in the non-sleep state.

In addition, the one or more processors 140 may, when the breathing sound is a sleep breathing sound, extract feature information of the sleep breathing sound. The one or more processors 140 may identify the user's sleep stage based on the extracted feature information.

Specifically, the one or more processors 140 may identify whether the breathing sound of the user corresponds to a breathing sound 22-2 in a first non-REM state, a breathing sound 22-3 in a second non-REM state, and a breathing sound 22-4 in a REM state according to the number of vibrations, wavelength, amplitude, intensity, etc. of the identified breathing sound of the user, and the one or more processors 140 may obtain information about a sleep state or a sleep stage corresponding to the type of each breathing sound.

In an embodiment, when it is identified that the posture of the user corresponds to a sleep posture based on the first reflection signal or the second reflection signal received via the communication interface 110, the one or more processors 140 may obtain brain wave information of the user. Here, the one or more processors 140 may perform a communication connection with the external server 200, an external device, or a user terminal device via the communication interface 110 to obtain brain wave information of the user. However, the embodiment is not limited thereto, and the one or more processors 140 may obtain brain wave information of the user based on the reflection signal received via the brainwave sensing communication interface 110 included in the electronic apparatus 100.

The one or more processors 140 may identify the feature information of the obtained brainwave information, and may obtain the information about the user's sleep state based on the identified user's posture, identified breathing sound, and the feature information of the brainwave information.

Here, the one or more processors 140 may control the aggregation & calculation module 150-3 to identify the sleep state of the user based on the information about the movement, posture, action, motion, or the like of the user obtained via the presense module 150-1 and the user's breathing sound identified via the sound module 150-2.

When the information about the sleep state or the sleep stage is obtained, the one or more processors 140 may transmit, to the external server 200 or external device, information about the sleep state of the user obtained by performing communication connection with the external server 200 or the external device via the communication interface 110 in operation S330.

In a state in which one or more processors 140 identify a user's posture based on a reflection signal received via the communication interface 110 and a sleep recognition function that identifies a user's breathing sound based on audio obtained via the microphone 120 is activated, the one or more processors 140, when the user's posture identified based on the obtained second reflection signal corresponds to a predetermined posture, for example, a posture corresponding to a non-sleep state, a walking posture, a standing posture, or a sitting posture, or a user's utterance is sensed for a predetermined time or more via the microphone 120, may activate the voice recognition function and deactivate the sleep recognition function.

That is, when the user's state becomes a non-sleep state from a sleep state based on the reflection signal received via the communication interface 110 or audio obtained via the microphone 120 in a state in which the sleep recognition function is activated, the voice recognition function of the electronic apparatus 100 may be activated again, and the sleep recognition function may be deactivated so that information, a service, and the like suitable for the current state of the user may be provided.

In the above embodiment, it has been described that one or more processors 140 activate a sleep recognition function of the electronic apparatus 100 according to whether the posture of the user corresponds to a sleep posture based on the reflection signal received via the communication interface 110, but as shown below, the sleep recognition function of the electronic apparatus 100 may be activated by identifying the movement frequency or the movement radius of the user based on the reflection signal received via the communication interface 110 as follows.

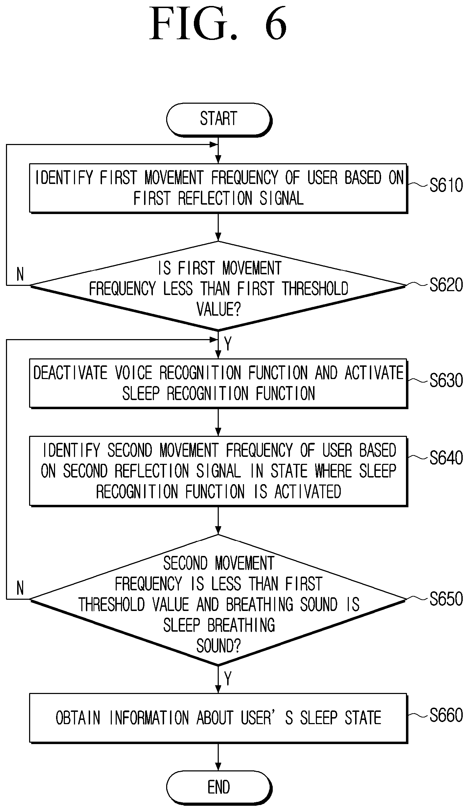

FIG. 6 is a flowchart illustrating an operation in which the electronic apparatus 100 deactivates a voice recognition function and activates a sleep recognition function by identifying a movement frequency of a user, according to an embodiment of the disclosure.

Referring to FIG. 6, one or more processors 140 may identify a first movement frequency of a user based on a first reflection signal received via the communication interface 110 in operation S610. Here, the movement of the user may mean that the position of the head, arm, leg, and body changes.

When the identified first movement frequency is less than the first threshold value in operation S620-Y, the one or more processors 140 may deactivate the voice recognition function and activate the sleep recognition function in operation S630.

In addition thereto, the one or more processors 140 may transmit, to the external server 200 via the communication interface 110, a first reflection signal for the posture, movement, and the like of the user received via the communication interface 110. The external server 200 may identify whether a movement frequency of the user is less than a first threshold value based on a first reflection signal received from the electronic apparatus 100. When it is identified that the user's movement frequency is less than the first threshold value in the external server 200, the one or more processors 140 may receive a control signal for deactivating a voice recognition function and activating a sleep recognition function from the external server 200 via the communication interface 110.

The one or more processors 140 may identify the second movement frequency of a user based on the second reflection signal received via the communication interface 110 in a state where the sleep recognition function is activated in operation S640.

When the second movement frequency is less than the first threshold value and the breathing sound is the sleep breathing sound in operation S650-Y, the one or more processor 140 may obtain the information about the user's sleep state in operation S660.

In addition, the one or more processors 140 may transmit, to the external server 200 via the communication interface 110, an audio obtained via the microphone 120 and a second reflection signal for the posture, movement, and the like of the user received via the communication interface 110 in a state in which the sleep recognition function is activated. The external server 200 may identify whether a movement frequency of the user is less than a first threshold value based on a second reflection signal received from the electronic apparatus 100, and identify a sleep state of the user by identifying a breathing sound of the user based on the audio received from the electronic apparatus 100. In this case, the one or more processors 140 may receive information about the sleep state and the sleep state of the user identified in the external server 200 via the communication interface 110.

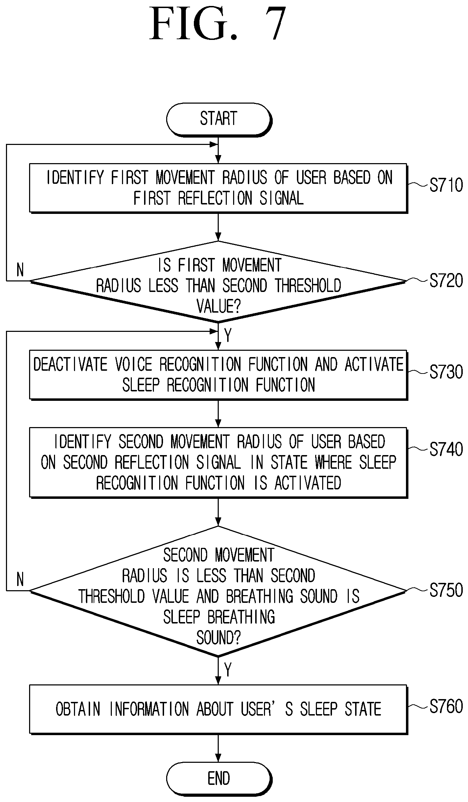

FIG. 7 is a flowchart illustrating an operation in which the electronic apparatus 100 deactivates a voice recognition function and activates a sleep recognition function by identifying a movement radius of a user, according to an embodiment of the disclosure.

Referring to FIG. 7, the one or more processors 140 may identify a first movement radius of a user based on a first reflection signal received via the communication interface 110 in operation S710. Here, the movement of the user may mean that the position of the head, the arm, the leg, and the body changes, and the movement radius may refer to a range in which the position of the head, the arm, the leg, and the body is changed or a range in which the body moves in the space, but is not limited thereto.

When the identified first movement radius is less than the second threshold value in operation S720-Y, the one or more processors 140 may deactivate the voice recognition function and may activate the sleep recognition function in operation S730.

In addition thereto, the one or more processors 140 may transmit, to the external server 200 via the communication interface 110, a first reflection signal for the posture, movement, and the like of the user received via the communication interface 110. The external server 200 may identify whether the movement radius of the user is less than a second threshold value based on the first reflection signal received from the electronic apparatus 100. When the external server 200 identifies that the user's movement radius is less than the second threshold value, the one or more processors 140 may receive a control signal for deactivating a voice recognition function and activating a sleep recognition function from the external server 200 via the communication interface 110.

The one or more processors 140 may identify the second movement radius of the user based on the second reflection signal received via the communication interface 110 in a state where the sleep recognition function is activated in operation S740.

When the second movement radius is less than the second threshold value and the breathing sound is the sleep breathing sound in operation S750-Y, the one or more processors 140 may obtain information about the user's sleep state in operation S760.

In addition, the one or more processors 140 may transmit, to the external server 200 via the communication interface 110, an audio obtained via the microphone 120 and a second reflection signal for the posture, movement, and the like of the user received via the communication interface 110 in a state in which the sleep recognition function is activated. The external server 200 may identify whether a movement radius of the user is less than a second threshold value based on a second reflection signal received from the electronic apparatus 100, and identify a sleep state of the user by identifying a breathing sound of the user based on the audio received from the electronic apparatus 100. In this case, the one or more processors 140 may receive the sleep state of the user identified in the external server 200 via the communication interface 110 and the information about the sleep state.

As described above, the one or more processors 140 may identify at least one of the posture of the user, the movement radius of the user, and the movement frequency of the user based on the reflection signal received via the communication interface 110. The one or more processors 140 may identify, based on the at least one of the identified posture of the user, the movement radius of the user, and the movement frequency of the user, whether at least one of the posture of the user, the movement radius, and the movement frequency of the user corresponds to the posture in the sleep state, the movement radius in the sleep state, and the movement frequency in the sleep state.

In addition, as described above, in the process in which the one or more processors 140 obtain information about the sleep state of the user, the audio or user's brain wave information obtained via the microphone 120 together with the reflection signal received via the communication interface 110 may be considered together.

FIG. 8 is a block diagram illustrating a configuration of the electronic apparatus 100 according to an embodiment of the disclosure.

Referring to FIG. 8, the electronic apparatus 100 may further include the sensor 150, the camera 160, the display 170, or the speaker 180, in addition to the communication interface 110, the microphone 120, the memory, and the one or more processor 140, or may omit some configurations.

The sensor 150 may be, for example, a gesture sensor, a proximity sensor, a motion sensor, or the like. In addition, the sensor 150 may be implemented as a Wi-Fi module, a Bluetooth module, a RADAR module, an IR module, a microwave module, a visible light sensor, an illuminance sensor, or the communication interface 110 included in the electronic apparatus 100.

The sensor 150 may configure a presense module for obtaining information about a movement (e.g., a movement radius, a movement frequency, a movement type, etc.), a posture, and a motion of a user based on the sensing result such as a movement (e.g., a movement radius, a movement frequency, a movement type, etc.), a posture, a motion, or the like, of a user located within a preset range from the electronic apparatus 100, but is not limited thereto, and the sensor 150 may be implemented as a device having a separate configuration from the presense module.

The sensor 150 may sense the presence, location, movement, posture, or the like, of the user located within the predetermined distance from the electronic apparatus 100 and may generate an electric signal or data value corresponding to the sensed state.

In the meantime, the sensor 150 is not limited thereto and may be various devices that may sense the posture, movement, motion, action, or the like, of the user.

The one or more processors 140 may identify whether the user's posture (e.g., walking posture, standing posture, sitting posture, crouching posture, lying posture, etc.) corresponds to the sleep posture based on the sensing result of the sensor 150. The one or more processors 140 may identify the frequency of motion of the user based on a sensing result of the sensor 150. The one or more processors 140 may identify a movement radius of the user based on a sensing result of the sensor 150.

Here, the user may be located in a place same as the electronic apparatus 100 or located within a preset distance from the electronic apparatus 100, but the embodiment is not limited thereto.

The one or more processors 140 may obtain a spectrogram corresponding to a sensing result of the sensor 150. The one or more processors 140 may obtain posture information of the user by inputting the obtained spectrogram into a state identification model (e.g., a neural network model).

The sensor 150 described above is described as sensing a motion (e.g., a movement radius, a movement frequency, a motion type, etc.) of a user located in a space same as the electronic apparatus 100 or located within a preset distance, a posture (e.g., a standing posture, a lying posture, a walking posture, a sitting posture, a sleep posture, etc.), a motion, etc., but the sensor 150 is not limited thereto, and may further include a biometric sensor (e.g., a heart rate sensor, a brainwave sensor, a blood glucose sensor, etc.) for obtaining biometric information of a user.

The camera 160 may be a device for capturing a still image and a video. According to an embodiment, the camera 160 may include a lens (e.g., a convex lens, a concave lens, a spherical lens, a planar lens, a wide-angle lens, etc.) which refracts or spreads one or more light; and an image sensor (for example, a charge-coupled device (CCD), a complementary metal-oxide semiconductor (CMOS)), an image signal one or more processors 140, or a flash. In addition, the camera 160 may include an iris, a viewfinder, a zebra device for sensing whether an exposure of an image through a CCD camera inside the camera 160 is excessive, and the like.

One or more processors 140 may obtain an RGB image by sensing light in a visible light region via the camera 160. The camera 160 may obtain an infrared image by sensing light in an infrared region. However, the embodiment is not limited thereto, and the one or more processors 140 may obtain an image by sensing light of various wavelength bands via the camera 160.

The one or more processors 140 may obtain an image by capturing a user's bed or a location, a place corresponding to the user's sleeping place.

The one or more processors 140 may identify whether the user is located in a bed or a sleeping place and is in a sleeping place based on the obtained location of a user's bed or a location corresponding to the user's sleeping place, and image of the space. At this time, the one or more processors 140 may input the obtained image to an image classification model (e.g., a neural network model) to identify whether the user located in the bed or the sleeping place is in a sleep state.

The one or more processors 140 may identify the posture of the user included in the image obtained via the camera 160. Specifically, the one or more processors 140 may obtain posture information of a user included in an image by inputting the obtained image to an image classification model.

The one or more processors 140 may obtain information about the sleep state of the user based on the identified user's posture, movement frequency, movement radius, and audio obtained via the microphone 120, based on the identified user's posture, movement frequency, movement radius, and audio obtained via the microphone 120, based on the user's posture identified based on the image obtained via the camera 160 and the reflection signal received via the communication interface 110.

According to various embodiments, one or more processors 140 may determine whether a blind spot exists. The one or more processors 140 may obtain an image (or a captured image) obtained via the camera 160. The one or more processors 140 may determine whether a user is included in a captured image. When a user is not included in the captured image, the one or more processors 140 may determine that there is a blind spot. When it is determined that the blind spot exists, the one or more processors 140 may perform various operations for capturing a blind spot.

For example, the one or more processors 140 may adjust an angle of view of the camera 160 to identify the user's movement, posture, or the like, of a user located at a blind spot of the camera 160.

For example, the one or more processors 140 may use an external camera connectable to the electronic apparatus 100 to identify a movement, a posture, and the like of a user located on a blind spot of the camera 160. The one or more processors 140 may transmit a control command for capturing a blind spot to an external camera. The one or more processors 140 may receive a captured image from an external camera. The one or more processors 140 may identify (or analyze) the movement, posture, and the like of the user based on the captured image received from the external camera. The external camera may be connected to the electronic apparatus 100 via the communication interface 110. For example, the external camera may be connected to the electronic apparatus 100 through a USB interface.

The display 170 may various types of display panels such as a liquid crystal display (LCD) panel, organic light emitting diodes (OLED) panel, active-matrix organic light-emitting diode (AM-OLED), liquid crystal on silicon (LcoS), quantum dot light-emitting diode (QLED), digital light processing (DLP), plasma display panel (PDP) panel, inorganic LED panel, micro LED panel, or the like, but is not limited thereto. In the meantime, the display 170 may configure a touch screen along with a touch panel and may be made of a flexible panel.

The display 170 may be implemented with a 2D-type square, rectangle, but is not limited thereto and may be implemented with various types such as a circular, polygonal, 3D type, or the like.

The display 170 may be disposed in one area of the surface of the electronic apparatus 100, but is not limited thereto, and may be a three-dimensional hologram display 170 projected on a space, and a projection display 170 projecting on a two-dimensional plane.

The one or more processors 140 may control the display 170 to output information about a sleep state of a user, a sleep stage of a user, or the like.

The one or more processors 140 may control the display 170 to output a GUI, image, and video for guiding a customized service operation corresponding to the sleep state or sleep stage of a user.

The one or more processors 140 may control the display 170 to output a GUI image, an image, and a video indicating whether the state of the current electronic apparatus 100 is a state in which the voice recognition function is activated or a state in which the sleep recognition function is activated.

The embodiment is not limited thereto, and the display 170 may be controlled to output a GUI, image, video to provide a user with various information related to an operation of identifying a posture of a user based on a reflection signal received via the communication interface 110, identifying a breathing sound of the user based on the audio obtained via the microphone 120, or an operation of obtaining information about a sleep state of a user based on the identified user's posture and the identified user's breathing sound.

The display 170 may be included as one configuration of the electronic apparatus 100, but is not limited thereto. A separately provided display 170 may be connected to the electronic apparatus 100 via the communication interface 110 or an input/output interface (not shown) through a wireless/wired manner to output an image, a video, and a GUI according to a signal of the one or more processors 140. In this case, the one or more processors 140 may perform connection with the display 170 in a wireless/wired manner via the communication interface 110 or an input/output interface (not shown) to transmit a signal for outputting an image, a video, and a GUI.

The speaker 180 may include a tweeter for playing a high-pitched sound; a mid-range for playing a mid-pitched sound; a woofer for playing a low-pitched sound; a subwoofer for playing a very low-pitched sound; an enclosure for controlling resonance; and a crossover network for dividing an electric signal frequency inputted to the speaker 180 for each band.

The speaker 180 may output the acoustic signal to the outside of the electronic apparatus 100. The speaker 180 may play multimedia, play recording, output various notification sounds, voice messages, and the like. The electronic apparatus 100 may include an audio output device such as the speaker 180, and may include an output device such as an audio output terminal. In particular, the speaker 180 may provide obtained information, processed and produced information, a response result or an operation result with respect to a user voice, or the like in a voice form based on the obtained information.

The one or more processors 140 may control the speaker 180 to output information about the user's sleep state, the information about the user's sleep stage, or the like.

The one or more processors 140 may control the speaker 180 to output voice to guide a customized service operation corresponding to the user's sleep state or sleep stage.

The one or more processors 140 may control the speaker 180 to output a voice indicating whether the current state of the electronic apparatus 100 is that the voice recognition function is activated or a sleep recognition function is activated.

The embodiment is not limited thereto, and the speaker 180 may be controlled to output a voice to provide a user with information related to an operation of identifying a posture of a user based on a reflection signal received via the communication interface 110, identifying a breathing sound of the user based on the audio obtained via the microphone 120, or obtaining information about the sleep state of the user based on the identified posture of the user and the identified breathing sound of the user.

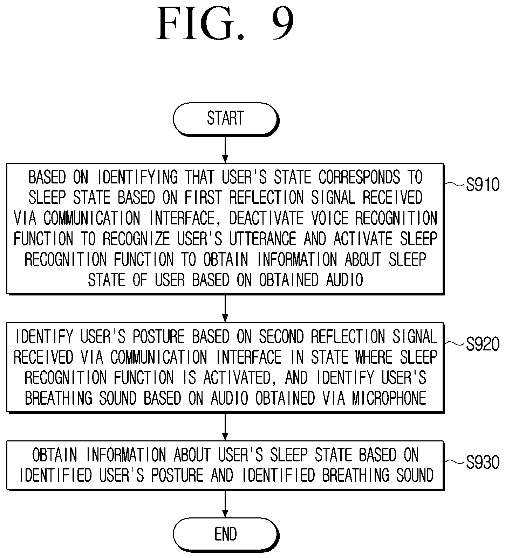

FIG. 9 is a flowchart illustrating an operation of the electronic apparatus 100 according to an embodiment of the disclosure.

Referring to FIG. 9, when it is identified that a posture of a user corresponds to a sleep posture (e.g., a lying posture, a crouching posture, etc.) based on the first reflection signal received via the communication interface 110, the electronic apparatus 100 may deactivate a voice recognition function for recognizing a user's utterance, and may activate a sleep recognition function for obtaining information about a sleep state of the user based on the obtained audio in operation S910.

In addition thereto, the one or more processors 140 may transmit, to the external server 200 via the communication interface 110, a first reflection signal for the posture, movement, and the like of the user received via the communication interface 110. The external server 200 may identify whether the posture of the user corresponds to the sleep posture based on the first reflection signal received from the electronic apparatus 100. When the external server 200 identifies that the posture of the user corresponds to the sleep posture, the one or more processors 140 may receive a control signal for deactivating a voice recognition function and activating a sleep recognition function from the external server 200 via the communication interface 110.

The electronic apparatus 100 may obtain a first spectrogram corresponding to the first reflection signal received via the communication interface 110. The electronic apparatus 100 may obtain first posture information of a user by inputting the obtained first spectrogram into a state identification model (e.g., a neural network model). The first spectrogram may have different waveforms according to a posture, a movement, a motion type, a movement frequency, a movement radius, and the like of a user.

When it is identified that the user's state corresponds to the sleep state (e.g., lying posture, crouching posture, etc.), the electronic apparatus 100 may deactivate the voice recognition function and activate the sleep recognition function.

The electronic apparatus 100 may identify the user's posture based on the second reflection signal received via the communication interface 110 in a state where the sleep recognition function is activated, and may identify the user's breathing sound based on the audio obtained via the microphone 120 in operation S920.

In addition, the electronic apparatus 100 may obtain a second spectrogram corresponding to the second reflection signal in a state in which the sleep recognition function is activated. The electronic apparatus 100 may obtain second posture information of the user by inputting the obtained second spectrogram into a state identification model (e.g., a neural network model). Here, the second reflection signal and the second spectrogram may have different waveforms according to the posture, movement, type of motion, movement frequency, movement radius, and the like of the user.

The electronic apparatus 100 may identify the user's posture based on the obtained information about the second posture and may identify the user's breathing sound based on the audio obtained via the microphone 120.

The electronic apparatus 100 may obtain information about the user's sleep state based on the identified user's posture and the identified breathing sound in operation S930.

In addition, the one or more processors 140 may transmit, to the external server 200 via the communication interface 110, an audio obtained via the microphone 120 and a second reflection signal for the posture, movement, and the like of the user received via the communication interface 110 in a state in which the sleep recognition function is activated. The external server 200 may identify whether the posture of the user corresponds to a sleep posture based on the second reflection signal received from the electronic apparatus 100, and identify a sleeping state of the user by identifying a breathing sound of the user based on the audio received from the electronic apparatus 100. In this case, the one or more processors 140 may receive the sleep state of the user identified in the external server 200 via the communication interface 110 and the information about the sleep state.

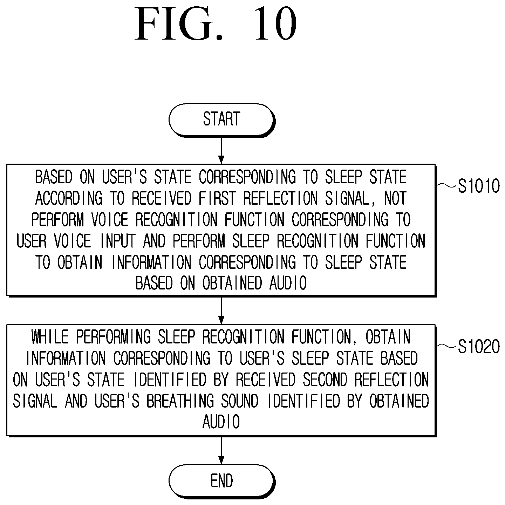

FIG. 10 is a flowchart illustrating an operation of an electronic apparatus according to an embodiment of the disclosure.

The control method of the electronic apparatus 100 may further include, based on identifying that a user's state corresponds to a sleep state based on a first reflection signal received via the communication interface, not performing a voice recognition function corresponding to a user voice input and performing a sleep recognition function to obtain information corresponding to the sleep state based on the obtained audio in operation S010; and while the sleep recognition function is being performed, obtaining information corresponding to the user's sleep state based on the user state identified by a second reflection signal and a breathing sound of the user identified by obtained audio in operation S1020.

The performing the sleep recognition function in operation S1010 may include obtaining first posture information of the user by inputting a first spectrogram obtained from the first reflection signal to a posture state identification model, based on the posture of the user corresponding to the sleep state according to the obtained first posture information of the user, not performing the voice recognition function and performing the sleep recognition function, and the obtaining the information corresponding to the sleep state of the user in operation S1020 may include, while performing the sleep recognition function, obtaining second posture information of the user by inputting a second spectrogram obtained from the second reflection signal to the posture state identification model, and identifying the posture of the user according to the obtained second posture information and identify the user's breathing sound based on obtained audio.

The performing the sleep recognition function in operation S1010 may include, based on a first movement frequency of the user based on the first reflection signal being less than a first threshold value, not performing the voice recognition function and performing the sleep recognition function, and the obtaining the information corresponding to the sleep state of the user in operation S1020 may include, while performing the sleep recognition function, based on a second movement frequency of the user based on the second reflection signal being less than a first threshold value and the user's breathing sound based on audio obtained via the microphone corresponding the sleep breathing sound, obtaining information corresponding to the user's sleep state.

The performing the sleep recognition function in operation S1010 may include, based on a first movement radius of the user based on the first reflection signal being less than a second threshold value, not performing the voice recognition function and performing the sleep recognition function, and the obtaining the information corresponding to the sleep state of the user in operation S1020 may include, while performing the sleep recognition function, based on the second movement radius of the user based on the second reflection signal being less than a second threshold value and the breathing sound of the user based on the audio obtained via the microphone corresponding to the sleep breathing sound, obtaining information corresponding to the user's sleep state.

The method may further include, while performing the sleep recognition function, based on the user's posture on the basis of the second reflection signal corresponding to a predetermined posture or receiving a user voice input for a predetermined time or more via the microphone, performing the voice recognition function and not performing the sleep recognition function, and the predetermined posture may include a walking posture, a standing posture, or a sitting posture.

The first reflection signal and the second reflection signal may include information corresponding to the user's posture comprising a walking posture, a standing posture, a sitting posture, a crouching posture, and a lying posture.

The obtaining the information corresponding to the user's sleep state in operation S1020 may include, based on the breathing sound corresponding to a sleep breathing sound, the one or more processors obtain information about the user's sleep stage based on feature information of the sleep breathing sound.

The obtaining the information corresponding to the user's sleep state in operation S1020 may include identifying the breathing sound based on data from which noise is removed, instead of the breathing sound, among audio obtained via the microphone.

The obtaining the information corresponding to the user's sleep state in operation S1020 may include, based on a posture of a user based on the first reflection signal corresponding to a sleep posture, obtaining brain wave information of the user, and obtaining information corresponding to the sleep state of the user based on the identified posture of the user, the identified breathing sound, and the obtained brain wave information.

The control method may include transmitting information corresponding to the obtained sleep state of the user to an external server or an external device.

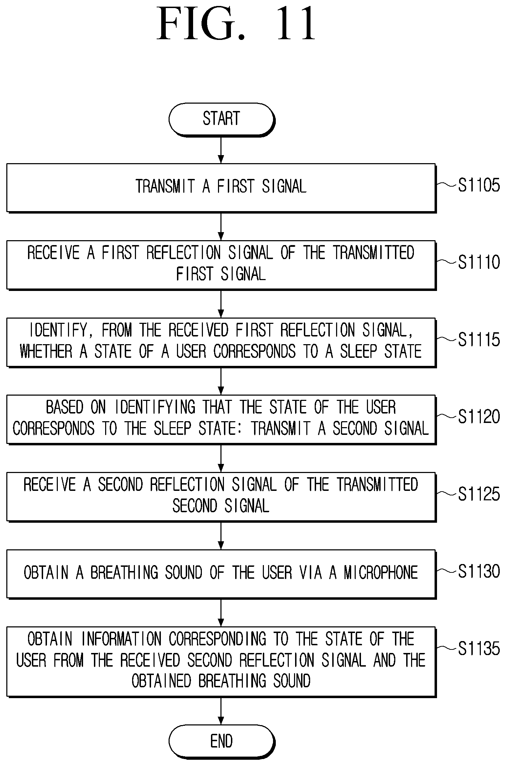

FIG. 11 is a flowchart illustrating an operation of an electronic apparatus according to an embodiment of the disclosure.

According to one or more embodiment, an electronic apparatus may include a memory storing at least one instruction and one or more processors configured to execute the at least one instruction to: transmit a first signal, receive a first reflection signal of the transmitted first signal, identify, from(or based on) the received first reflection signal, whether a state of a user corresponds to a sleep state, and based on identifying that the state of the user corresponds to the sleep state: transmit a second signal, receive a second reflection signal of the transmitted second signal, obtain a breathing sound of the user via a microphone, and obtain information corresponding to the state of the user from the received second reflection signal and the obtained breathing sound.

The one or more processors may execute the at least one instruction to: obtain a first spectrogram from the received first reflection signal, obtain first posture information of the user from the obtained first spectrogram, identify whether the obtained first posture information corresponds to the sleep state, and based on identifying that the first posture information corresponds to the sleep state: obtain a second spectrogram from the received second reflection signal, obtain second posture information of the user from the obtained second spectrogram, and identify a posture of the user from the obtained second posture information and the obtained breathing sound.

The one or more processors may execute the at least one instruction to: obtain a first movement frequency of the user from the received first reflection signal, and based on the obtained first movement frequency being less than a first threshold value, obtain a second movement frequency of the user from the received second reflection signal, wherein the information corresponding to the state of the user is obtained based on the obtained second movement frequency being less than the first threshold value and the obtained breathing sound corresponding to a sleep breathing sound.

The one or more processors may execute the at least one instruction to: obtain a first movement radius of the user from the received first reflection signal, and based on the obtained first movement radius being less than a threshold value obtain a second movement radius of the user from the received second reflection signal, wherein the information corresponding to the state of the user is obtained based on the obtained second movement radius being less than the threshold value and the obtained breathing sound corresponding to a sleep breathing sound.

The transmitting of the second signal, the receiving of the second reflection signal, the obtaining of the breathing sound, and the obtaining of the information corresponding to the state of the user may be performed as a sleep recognition function, and the one or more processors may execute the at least one instruction to: obtain a second spectrogram from the received second reflection signal, obtain second posture information of the user from the obtained second spectrogram, identify a posture of the user from the obtained second posture information, and based on the identified posture of the user corresponding to a predetermined posture or a voice input being received from the user for at least a predetermined time via the microphone: perform a voice recognition function, and deactivate the sleep recognition function, wherein the predetermined posture includes a walking posture, a standing posture, and a sitting posture.

The received first reflection signal and the received second reflection signal include information corresponding to a posture of the user including a walking posture, a standing posture, a sitting posture, a crouching posture, and a lying posture.

Based on the obtained breathing sound corresponding to a sleep breathing sound, the one or more processors is further configured to execute the at least one instruction to: obtain information about a sleep stage of the user based on feature information of the sleep breathing sound. The sleep stage includes a non-sleep stage, a non-rapid eye movement (REM) sleep stage, and an REM stage.

The one or more processors may execute the at least one instruction to: remove noise from the obtained breathing sound to form noise-removed audio data, and obtain the breathing sound from the noise-removed audio data.

The one or more processors may execute the at least one instruction to: obtain a first spectrogram from the received first reflection signal, obtain first posture information of the user from the obtained first spectrogram, identify a posture of the user from the obtained first posture information, and based on the identified first posture information corresponding to a sleep posture, obtain brain wave information of the user. The information corresponding to the state of the user is obtained based on the identified posture of the user, the obtained breathing sound, and the obtained brain wave information.

The one or more processors may execute the at least one instruction to: transmit the obtained information corresponding to the state of the user to an external server or an external device.

According to one or more embodiment, a control method of an electronic apparatus, the method may include transmitting a first signal (S1105), receiving a first reflection signal of the transmitted first signal (S1110), identifying, from the received first reflection signal, whether a state of a user corresponds to a sleep state (S1115), and based on identifying that the state of the user corresponds to the sleep state: transmitting a second signal (S1120), receiving a second reflection signal of the transmitted second signal (S1125), obtaining a breathing sound of the user via a microphone (S1130), and obtaining information corresponding to the state of the user from the received second reflection signal and the obtained breathing sound (S1135).

The method may include obtaining a first spectrogram from the received first reflection signal, obtaining first posture information of the user from the obtained first spectrogram, identifying whether the obtained first posture information corresponds to the sleep state, and based on identifying that the first posture information corresponds to the sleep state: obtaining a second spectrogram from the received second reflection signal, obtaining second posture information of the user from the obtained second spectrogram, and identifying a posture of the user from the obtained second posture information and the obtained breathing sound.

The method may include obtaining a first movement frequency of the user from the first reflection signal, and based on the obtained first movement frequency being less than a first threshold value: obtaining a second movement frequency of the user from the received second reflection signal. The information corresponding to the state of the user is based on the obtained second movement frequency being less than the first threshold value and the obtained breathing sound.

The method may include obtaining a first movement radius of the user from the received first reflection signal, and based on the obtained first movement radius being less than a threshold value, obtaining a second movement radius of the user from the received second reflection signal. The information corresponding to the state of the user is based on the obtained second movement radius being less than the threshold value and the obtained breathing sound corresponding to a sleep breathing sound.

The transmitting the second signal, the receiving the second reflection signal, the obtaining the breathing sound, and the obtaining the information corresponding to the state of the user may be performed as a sleep recognition function. The method may include obtaining a second spectrogram from the received second reflection signal, obtaining second posture information of the user from the obtained second spectrogram, identifying a posture of the user from the obtained second posture information, and based on the identified posture of the user corresponding to a predetermined posture or receiving a voice input from the user for at least a predetermined time: performing a voice recognition function, and deactivating the sleep recognition function. The predetermined posture includes a walking posture, a standing posture, and a sitting posture.

According to an embodiment, the methods according to various embodiments herein may be provided in a computer program product. A computer program product may be exchanged between a seller and a purchaser as a commodity. A computer program product may be distributed in the form of a machine-readable storage medium (e.g., compact disc read only memory (CD-ROM)) or distributed online through an application store (e.g. PlayStore ^{™}) directly between two user devices (e.g., smartphones). In the case of on-line distribution, at least a portion of the computer program product may be stored temporarily or at least temporarily in a storage medium such as a manufacturer's server, a server of an application store, or a memory of a relay server.

While various embodiments have been illustrated and described with reference to various embodiments, the disclosure is not limited to specific embodiments or the drawings, and it will be understood by those of ordinary skill in the art that various changes in form and details may be made therein without departing from the spirit and scope of the disclosure, including the appended claims and their equivalents.

## Claims

1. An electronic apparatus comprising:
a communication interface;
a microphone;
a memory storing at least one instruction; and
one or more processors connected to the communication interface, the microphone, and the memory and configured to the electronic apparatus,
wherein the one or more processors are configured to:
based on identifying that a user's state corresponds to a sleep state according to a first reflection signal received via the communication interface, not perform a voice recognition function corresponding to a user voice input and perform a sleep recognition function to obtain information corresponding to the sleep state based on the obtained audio, and
while the sleep recognition function is being performed, obtain information corresponding to the user's sleep state based on the user state identified by a second reflection signal received via the communication interface and a breathing sound of the user identified by audio obtained via the microphone.

2. The electronic apparatus of claim 1, wherein the one or more processors are configured to:
obtain first posture information of the user by inputting a first spectrogram obtained from the first reflection signal to a posture state identification model,
based on the posture of the user corresponding to the sleep state according to the obtained first posture information of the user, not perform the voice recognition function and perform the sleep recognition function,
while performing the sleep recognition function, obtain second posture information of the user by inputting a second spectrogram obtained from the second reflection signal to the posture state identification model, and
identify the posture of the user according to the obtained second posture information and identify the user's breathing sound based on audio obtained via the microphone.

3. The electronic apparatus of claim 1, wherein the one or more processors are configured to:
based on a first movement frequency of the user according to the first reflection signal being less than a first threshold value, not perform the voice recognition function and perform the sleep recognition function, and
while performing the sleep recognition function, based on a second movement frequency of the user according to the second reflection signal being less than a first threshold value and the user's breathing sound based on audio obtained via the microphone corresponding the sleep breathing sound, obtain information corresponding to the user's sleep state.

4. The electronic apparatus of claim 1, wherein the one or more processors are configured to:
based on a first movement radius of the user according to the first reflection signal being less than a second threshold value, not perform the voice recognition function and perform the sleep recognition function,
while performing the sleep recognition function, based on the second movement radius of the user according to the second reflection signal being less than a second threshold value and the breathing sound of the user based on the audio obtained via the microphone corresponding to the sleep breathing sound, obtain information corresponding to the user's sleep state.

5. The electronic apparatus of claim 1, wherein the one or more processors are configured to, while performing the sleep recognition function, based on the user's posture on the basis of the second reflection signal corresponding to a predetermined posture or receiving a user voice input for a predetermined time or more via the microphone, perform the voice recognition function and not perform the sleep recognition function, and
wherein the predetermined posture comprises a walking posture, a standing posture, or a sitting posture.

6. The electronic apparatus of claim 1, wherein the first reflection signal and the second reflection signal comprise information corresponding to the user's posture comprising a walking posture, a standing posture, a sitting posture, a crouching posture, and a lying posture.

7. The electronic apparatus of claim 1, wherein, based on the breathing sound corresponding to a sleep breathing sound, the one or more processors obtain information about the user's sleep stage based on feature information of the sleep breathing sound,
wherein the sleep stage comprises a non-sleep stage, a non-rapid eye movement (REM) sleep stage, and an REM stage.

8. The electronic apparatus of claim 1, wherein the one or more processors are configured to identify the breathing sound based on data from which noise is removed, instead of the breathing sound, among audio obtained via the microphone.

9. The electronic apparatus of claim 1, wherein the one or more processors are configured to:
based on a posture of a user according to the first reflection signal corresponding to a sleep posture, obtain brain wave information of the user, and
obtain information corresponding to the sleep state of the user based on the identified posture of the user, the identified breathing sound, and the obtained brain wave information.

10. The electronic apparatus of claim 1, wherein the one or more processors are configured to transmit the information corresponding to the obtained sleep state of the user to an external server or an external device connected via the communication interface.

11. A control method of an electronic apparatus, the method further comprising:
based on identifying that a user's state corresponds to a sleep state according to a first reflection signal received via the communication interface, not performing a voice recognition function corresponding to a user voice input and performing a sleep recognition function to obtain information corresponding to the sleep state based on the obtained audio; and
while the sleep recognition function is being performed, obtaining information corresponding to the user's sleep state based on the user state identified by a second reflection signal and a breathing sound of the user identified by obtained audio.

12. The method of claim 11, wherein the performing the sleep recognition function comprises:
obtaining first posture information of the user by inputting a first spectrogram obtained from the first reflection signal to a posture state identification model,
based on the posture of the user corresponding to the sleep state according to the obtained first posture information of the user, not performing the voice recognition function and performing the sleep recognition function,
wherein the obtaining the information corresponding to the sleep state of the user comprises:
while performing the sleep recognition function, obtaining second posture information of the user by inputting a second spectrogram obtained from the second reflection signal to the posture state identification model, and
identifying the posture of the user according to the obtained second posture information and identify the user's breathing sound based on obtained audio.

13. The method of claim 11, wherein the performing the sleep recognition function comprises, based on a first movement frequency of the user according to the first reflection signal being less than a first threshold value, not performing the voice recognition function and performing the sleep recognition function,
wherein the obtaining the information corresponding to the sleep state of the user comprises, while performing the sleep recognition function, based on a second movement frequency of the user according to the second reflection signal being less than a first threshold value and the user's breathing sound based on audio obtained via the microphone corresponding the sleep breathing sound, obtaining information corresponding to the user's sleep state.

14. The method of claim 11, wherein the performing the sleep recognition function comprises, based on a first movement radius of the user according to the first reflection signal being less than a second threshold value, not performing the voice recognition function and performing the sleep recognition function,
wherein the obtaining the information corresponding to the sleep state of the user comprises, while performing the sleep recognition function, based on the second movement radius of the user according to the second reflection signal being less than a second threshold value and the breathing sound of the user based on the audio obtained via the microphone corresponding to the sleep breathing sound, obtaining information corresponding to the user's sleep state.

15. The method of claim 11, further comprising:
while performing the sleep recognition function, based on the user's posture on the basis of the second reflection signal corresponding to a predetermined posture or receiving a user voice input for a predetermined time or more via the microphone, performing the voice recognition function and not performing the sleep recognition function, and
wherein the predetermined posture comprises a walking posture, a standing posture, or a sitting posture.
